# EUROPÄISCHE PATENTSCHRIFT

(11) **EP 2 007 773 B1**
(45) Veröffentlichungstag und Bekanntmachung des Hinweises auf die Patenterteilung: **24.10.2012**
(21) Anmeldenummer: 07727564.2
(22) Anmeldetag: 30.03.2007
(51) Int. Cl.: C07D 513/04, A61K 31/429, A61P 35/00

(54) **THIAZOLYL-DIHYDRO-INDAZOLE**
THIAZOLYL DIHYDRO-INDAZOLES
THIAZOLYL-DIHYDRO-INDAZOLES

(30) Priorität: 06.04.2006 EP 06112303
(43) Veröffentlichungstag der Anmeldung: 31.12.2008
(73) Patentinhaber: Boehringer Ingelheim International GmbH, 55216 Ingelheim am Rhein (DE)
(72) Erfinder: STEURER, Steffen, A-1190 Vienna (AT); BETZEMEIER, Bodo, A-2721 Bad Fischau (AT); MCCONNELL, Darryl, A-1130 Vienna (AT); GERSTBERGER, Thomas, A-1230 Vienna (AT); GRAUERT, Matthias, 88400 Biberach (DE); HOFFMANN, Matthias, 88441 Mittelbiberach (DE); IMPAGNATIELLO, Maria, A-1030 Vienna (AT); VAN DER VEEN, Lars, A-1230 Vienna (AT); WEYER-CZERNILOFSKY, Ulrike, A-2500 Baden (AT)
(74) Vertreter: Simon, Elke Anna Maria
(86) Internationale Anmeldenummer: PCT/EP2007/053092
(87) Internationale Veröffentlichungsnummer: WO 2007/113245

(56) Entgegenhaltungen:
- WO-A-01/57008
- WO-A-2006/040281
- DE-A1-102004 048 877

## Beschreibung

Die vorliegende Erfindung betrifft neue Thiazolyl-dihydro-indazole der allgemeinen Formel (1) 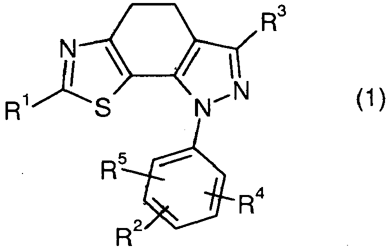 wobei die Reste R¹ bis R⁵ die in den Ansprüchen und der Beschreibung genannten Bedeutungen haben, deren Isomere, Verfahren zur Herstellung dieser Thiazolyl-dihydro-indazole sowie deren Verwendung zur Herstellung eines Arzneimittels.

### Hintergrund der Erfindung

Die Phosphorylierung von Proteinen und Lipiden ist ein wichtiger zellulärer Regulationsmechanismus, der bei multiplen biologischen Vorgängen, wie z.B. Zellproliferation, Differenzierung, Apoptose, Metabolismus, Inflammation, Immunreaktionen und Angiogenese eine Rolle spielt. Im humanen Genom sind über 500 Kinasen kodiert. Tyrosin-Protein-Kinasen werden im allgemeinen durch Wachstumsfaktoren oder andere mitogene Signale stimuliert und phosphorylieren Proteine, die schnelle Signalübertragungen initiieren. Serin/Threonin-Protein-Kinasen phosphorylieren meist Proteine, die intrazelluläre Signale vernetzen und amplifizieren. Lipid-Kinasen sind ebenfalls wichtige Schaltstellen intrazellulärer Signalwege, die diverse biologische Vorgänge verknüpfen.

Eine Reihe von Protein-Kinasen haben sich bereits als geeignete Zielmoleküle für die therapeutische Intervention in verschiedenen Indikationen, z.B. Krebs, entzündlichen und Autoimmunerkrankungen erwiesen. Da ein hoher Prozentsatz der bisher identifizierten, an der Krebsentstehung beteiligten Gene für Kinasen kodiert, stellen diese Enzyme speziell für die Krebstherapie attraktive Zielmoleküle dar.

Wo 01/57008 offenbart 4,5 - Dihydro - pyrazolo [4,3-9] benzothiazol - Verbindungen, welche als Inhibitoren von spezifischen Zellzykluskinasen verwendet werden.

### Detaillierte Beschreibung der Erfindung

Es wurde nun überraschenderweise gefunden, dass Verbindungen der allgemeinen Formel (1), worin die Reste R¹ bis R⁵ die nachstehend genannten Bedeutungen haben, als Inhibitoren spezifischer Zellzykluskinasen wirken. Somit können die erfindungsgemäßen Verbindungen beispielsweise zur Behandlung von Erkrankungen, die mit der Aktivität spezifischer Zellzykluskinasen in Zusammenhang stehen und durch exzessive oder anomale Zellproliferation charakterisiert sind, verwendet werden.

Die vorliegende Erfindung betrifft Verbindungen der allgemeinen Formel (1) 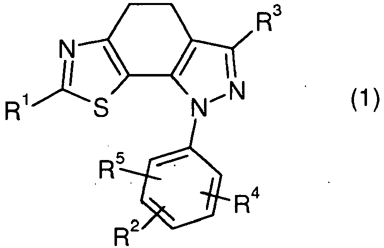 worin
R¹ ausgewählt aus der Gruppe bestehend aus -NHC(O)OR^{c}, und
R² C₁₋₆Alkyl oder 3-8 gliedriges Heterocycloalkyl, gegebenenfalls substituiert mit einem oder mehreren R⁶, oder ein Rest ausgewählt aus der Gruppe bestehend aus Halogen, -NO₂, -NR^{c}R^{c}, -OR^{c}, - S(O)R^{c}, -S(O)₂R^{c}, -C(O)R^{c}, -C(O)OR^{c}, -C(O)NR^{c}R^{c}, -C(O)N(R^{g})OR^{c} -N(R^{g})C(O)R^{c}, -N(R^{g})C(O)OR^{c}, -NR^{g}C(O)NR^{c}R^{c}-N(R^{g})C(O)SR^{c} und -N(R^{g})S(O)₂R^{c}, und R³ ein Rest ausgewählt aus der Gruppe bestehend aus C₆₋₁₀Aryl und 5-6 gliedriges Heteroaryl, gegebenenfalls substituiert mit einem oder mehreren, gleich oder verschiedenen R^{c} und/oder R^{b}, und
R⁴ ein Rest ausgewählt aus der Gruppe bestehend aus Brom, Fluor, -CF₃, -OCF₃, -CN, -NR^{c}R^{c}, -SR^{c}, -S(O)R^{c}, -S(O)₂R^{c} und -OR^{c}, oder C₁₋₃Alkyl gegebenenfalls substituiert mit Fluor, -CN, -NR^{f}R^{f} und/oder -OR^{f}, und
R⁵ Wasserstoff oder ein Rest ausgewählt aus der Gruppe bestehend aus Halogen, -CF₃, - OCF₃, -CN, -NR^{c}R^{c}, -SR^{c}, -S(O)R^{c}, -S(O)₂R^{c} und -OR^{c}, oder C₁₋₃Alkyl gegebenenfalls substituiert mit Fluor, -CN, -NR^{f}R^{f} und/oder -OR^{f}, und
R⁶ ein Rest ausgewählt aus der Gruppe bestehend aus R^{a}, R^{b} und R^{a} substituiert mit einem oder mehreren, gleich oder verschiedenen R^{c} und/oder R^{b}, und
jedes R^{a} unabhängig voneinander ausgewählt aus der Gruppe bestehend aus C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl, und
jedes R^{b} ein geeigneter Rest und jeweils unabhängig voneinander ausgewählt aus der Gruppe bestehend aus =O, -OR^{c}, C₁₋₃Haloalkyloxy, -OCF₃, =S, -SR^{c}, =NR^{c}, =NOR^{c}, -NR^{c}R^{c}, Halogen, -CF₃, -CN, -NC, -NO₂, -N₃, -S(O)R^{c}, -S(O)₂R^{c}, -S(O)₂OR^{c}, -S(O)NR^{c}R^{c}, -S(O)₂NR^{c}R^{c}, -OS(O)R^{c}, -OS(O)₂R^{c}, -OS(O)₂OR^{c}, -OS(O)₂NR^{c}R^{c}, -C(O)R^{c}, -C(O)OR^{c}, -C(O)NR^{c}R^{c}, -C(O)N(R^{g})NR^{c}R^{c}, -C(O)N(R^{g})OR^{c}, -CN(R^{g})NR^{c}R^{c}, -OC(O)R^{c}, -OC(O)OR^{c}, -OC(O)NR^{c}R^{c}, -N(R^{g})C(O)R^{c}, -N(R^{g})C(O)R^{c}, -N(R^{g})C(S)R^{c}, -N(R^{g})S(O)₂R^{c}, -N(R^{g})S(O)₂NR^{c}R^{c}, -N(R^{g})C(O)OR^{c}, -N(R^{g})C(O)NR^{c}R^{c}, und -N(R^{g})CN(R^{g})NR^{c}R^{c}, und jedes R^{c} unabhängig voneinander Wasserstoff oder ein gegebenenfalls mit einem oder mehreren, gleich oder verschiedenen R^{d} und/oder R^{e} substituierter Rest ausgewählt aus der Gruppe bestehend aus C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl, jedes R^{d} unabhängig voneinander ein gegebenenfalls mit einem oder mehreren, gleich oder verschiedenen R^{e} und/oder R^{f} substituierter Rest ausgewählt aus der Gruppe bestehend aus C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl, und
jedes R^{e} ein geeigneter Rest und jeweils unabhängig voneinander ausgewählt aus der Gruppe bestehend aus =O, -OR^{f}, C₁₋₃Haloalkyloxy, -OCF₃, =S, -SR^{f}, =NR^{f}, =NOR^{f}, -NR^{f}R^{f}, Halogen, -CF₃, -CN, -NC, -NO₂, -N₃, -S(O)R^{f}, -S(O)₂R^{f}, -S(O)₂OR^{f}, -S(O)NR^{f}R^{f}, - S(O)₂NR^{f}R^{f}, -OS(O)R^{f}, -OS(O)₂R^{f}, -OS(O)₂OR^{f}, -OS(O)₂NR^{f}R^{f}, -C(O)R^{f}, -C(O)OR^{f}, -C(O)NR^{f}R^{f}, -C(O)N(R^{g})OR^{f} -CN(R^{g})NR^{f}R^{f}, -OC(O)R^{f}, -OC(O)OR^{f}, -OC(O)NR^{f}R^{f}, -OCN(R^{g})NR^{f}R^{f}, -N(R^{g})C(O)R^{f}, -N(R^{g})C(S)R^{f}, -N(R^{g})S(O)₂R^{f}, -N(R^{g})C(O)OR^{f}, -N(R^{g})C(O)NR^{f}R^{f}, und -N(R^{g})CN(R^{g})NR^{f}R^{f}, und
jedes R^{f} unabhängig voneinander Wasserstoff oder ein gegebenenfalls mit einem oder mehreren, gleich oder verschiedenen R^{g} substituierter Rest ausgewählt aus der Gruppe bestehend aus C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl, und jedes R^{g} unabhängig voneinander Wasserstoff, C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl bedeuten, gegebenenfalls in Form ihrer Tautomeren, ihrer Racemate, ihrer Enantiomere, ihrer Diastereomere und ihrer Gemische, sowie gegebenenfalls ihrer pharmakologisch verträglichen Salze

Ein Aspekt der Erfindung sind Verbindungen der allgemeinen Formel (1A), 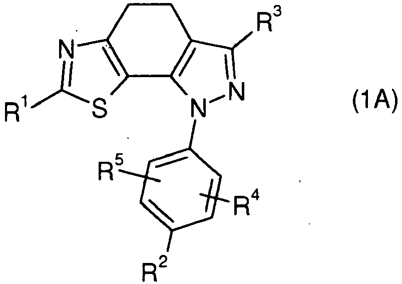 worin die Substituenten wie vorstehend definiert sind.

Ein Aspekt der Erfindung sind Verbindungen der allgemeinen Formel (1B), 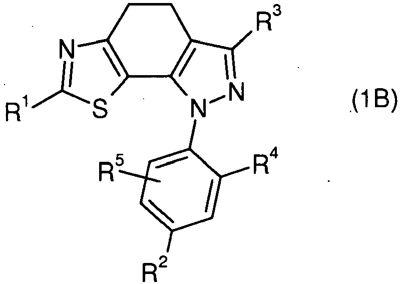 worin die Substituenten wie vorstehend definiert sind.

Ein Aspekt der Erfindung sind Verbindungen der allgemeinen Formel (1), (1A) oder (1B), worin R³ 5-6 gliedriges Heteroaryl, gegebenenfalls substituiert mit einem oder mehreren, gleich oder verschiedenen R^{c} und/oder R^{b} bedeutet.

Ein Aspekt der Erfindung sind Verbindungen der allgemeinen Formel (1), (1A) oder (1B); worin R³ unsubstituiertes Pyridyl bedeutet.

Ein Aspekt der Erfindung sind Verbindungen der allgemeinen Formel (1), (1A) oder (1B), worin R¹ ausgewählt ist aus der Gruppe bestehend aus -NHC(O)OR^{c}.
(A) Aspekte bezüglich R¹
   (A1) Ein Aspekt der Erfindung sind Verbindungen der allgemeinen Formel (1), (1A) oder (1B), worin R¹ -NHC(O)OCH₃ bedeutet.
(B) Aspekte bezüglich R²
   (B1) Ein Aspekt der Erfindung sind Verbindungen der allgemeinen Formel (1), (1A) oder (1B), worin R² -C(O)NR^{c}R^{c} bedeutet.
   (B2) Ein weiterer Aspekt der Erfindung sind Verbindungen der allgemeinen Formel (1), (1A) oder (1B), worin R² -C(O)NHR^{c} und R^{c} Methyl, C₁₋₃Alkyl, gegebenenfalls substituiert mit -OR^{f}, -NR^{f}R^{f}, 3-8 gliedriges Heterocycloalkyl, gegebenenfalls substituiert mit Methyl, bedeutet.
   (B3) Ein weiterer Aspekt der Erfindung sind Verbindungen der allgemeinen Formel (1), (1A) oder (1B), worin R² -C(O)N(CH₃)R^{c} und R^{c} Methyl, C₁₋₃Alkyl, gegebenenfalls substituiert mit OR^{f} oder NR^{f}R^{f} oder Heterocycloalkyl, gegebenenfalls substituiert mit Methyl, bedeutet.
   (B4) Ein weiterer Aspekt der Erfindung sind Verbindungen der allgemeinen Formel (1), (1A) oder (1B), worin R² -C(O)R^{c} und R^{c} 3-8 gliedriges Heterocycloalkyl, gegebenenfalls substituiert mit C₁₋₃Alkyl oder -NR^{f}R^{f}, 5-6 gliedriges Heteroaryl oder (5-6 gliedriges Heteroaryl)-methyl bedeutet.
   (B5) Ein weiterer Aspekt der Erfindung sind Verbindungen der allgemeinen Formel (1), (1A) oder (1B), worin R² -NHC(O)R^{c} und R^{c} C₁₋₄Alkyl, gegebenenfalls substituiert mit -OR^{f} oder -NR^{f}R^{f}, 3-8 gliedriges Cycloalkyl, 3-8 gliedriges Heterocycloalkyl, gegebenenfalls substituiert mit Methyl oder 5-6 gliedriges Heteroaryl, gegebenenfalls substituiert mit Methyl, bedeutet.
   (B6) Ein weiterer Aspekt der Erfindung sind Verbindungen der allgemeinen Formel (1), (1A) oder (1B), worin R² -NHC(O)OR^{c} und R^{c} C₁₋₄Alkyl, gegebenenfalls substituiert mit -OR^{f} oder -NR^{f}R^{f}, 3-8 gliedriges Cycloalkyl, 3-8 gliedriges Heterocycloalkyl, gegebenenfalls substituiert mit Methyl oder 5-6 gliedriges Heteroaryl, gegebenenfalls substituiert mit Methyl, bedeutet.
(C) Aspekte bezüglich R⁴
   (C1) Ein Aspekt der Erfindung sind Verbindungen der allgemeinen Formel (1), (1A) oder (1B), worin R⁴ Fluor bedeutet.
   (C2) Ein weiterer Aspekt der Erfindung sind Verbindungen der allgemeinen Formel (1), (1A) oder (1B), worin R⁴ Brom bedeutet.
(D) Aspekte bezüglich R⁵
   (D1) Ein Aspekt der Erfindung sind Verbindungen der allgemeinen Formel (1), (1A) oder (1B), worin R⁵ Wasserstoff bedeutet.
   (D2) Ein Aspekt der Erfindung sind Verbindungen der allgemeinen Formel (1), (1A) oder (1B), worin R⁵ Fluor bedeutet.

Ein Aspekt der Erfindung sind Verbindungen der allgemeinen Formel (1), (1A) oder (1B), oder deren pharmazeutisch wirksamen Salze, als Arzneimittel.

Ein Aspekt der Erfindung sind Verbindungen der allgemeinen Formel (1), (1A) oder (1B), oder deren pharmakologisch verträglichen Salze, zur Herstellung eines Arzneimittels mit antiproliferativer Wirkung.

Ein Aspekt der Erfindung ist eine pharmazeutische Zubereitungen, enthaltend als Wirkstoff eine oder mehrere Verbindungen der allgemeinen Formel (1), (1A) oder (1B), oder deren pharmakologisch verträglichen Salze, gegebenenfalls in Kombination mit üblichen Hilfs- und/oder Trägerstoffen.

Ein Aspekt der Erfindung ist die Verwendung von Verbindungen der allgemeinen Formel (1), (1A) oder (1B) zur Herstellung eines Arzneimittels zur Behandlung und/oder Prävention von Krebs.

Ein Aspekt der Erfindung ist eine pharmazeutische Präparation umfassend eine Verbindung der allgemeinen Formel (1), (1A) oder (1B) und mindestens eine weitere zytostatische oder zytotoxische, von Formel (1), (1A) oder (1B) verschiedene Wirksubstanz, gegebenenfalls in Form ihrer Tautomeren, ihrer Racemate, ihrer Enantiomere, ihrer Diastereomere und ihrer Gemische, sowie gegebenenfalls ihrer pharmakologisch verträglichen Salze.

### Definitionen

Wie hierin verwendet treffen folgenden Definitionen zu, falls nicht anders beschrieben.

Unter Alkyl-Substitutenten sind jeweils gesättigte, ungesättigte, geradkettige oder verzweigte aliphatische Kohlenwasserstoffreste (Alkylrest) zu verstehen und umfasst sowohl gesättigte Alkylreste als auch ungesättigte Alkenyl- und Alkinylreste. Alkenyl-Substituenten sind jeweils geradkettige oder verzweigte, ungesättigte Alkylreste, die mindestens eine Doppelbindung aufweisen. Unter Alkinyl-Substituenten sind jeweils geradkettige oder verzweigte, ungesättigte Alkylreste, die mindestens eine Dreifachbindung aufweisen; zu verstehen.

Durch den Ausdruck Heteroalkyl werden Reste repräsentiert, die sich aus vorstehend definiertem Alkyl in seiner weitesten Bedeutung dadurch ableiten, dass in den Kohlenwasserstoffketten eine oder mehrere der Gruppen -CH₃ unabhängig voneinander durch die Gruppen -OH, -SH oder -NH₂, eine oder mehrere der Gruppen -CH₂- unabhängig voneinander durch die Gruppen -O,- -S- oder -NH-, eine oder mehrere der Gruppen 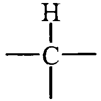 durch die Gruppe 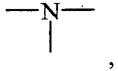 eine oder mehrere der Gruppen =CH- durch die Gruppe =N-, eine oder mehrere der Gruppen =CH₂ durch die Gruppe =NH oder eine oder mehrere der Gruppen ≡CH durch die Gruppe ≡N ersetzt werden, wobei insgesamt nur maximal drei Heteroatome in einem Heteroalkyl vorhanden sein können, zwischen zwei Sauerstoff- und zwischen zwei Schwefelatomen bzw. zwischen je einem Sauerstoff- und einem Schwefelatom mindestens ein Kohlenstoffatom vorliegen und der Rest insgesamt über chemische Stabilität verfügen muss.

Aus der indirekten Definition/Ableitung aus Alkyl ergibt sich unmittelbar, dass sich Heteroalkyl aus den Untergruppen gesättigte Kohlenwasserstoffketten mit Heteroatom(en), Heteroalkenyl und Heteroalkinyl zusammensetzt, wobei eine weitere Unterteilung in geradkettig (unverzweigt) und verzweigt vorgenommen werden kann. Sollte ein Heteroalkyl substituiert sein, so kann die Substitution unabhängig voneinander, jeweils ein- oder mehrfach, an allen wasserstofftragenden Sauerstoff-, Schwefel-, Stickstoff- und/oder Kohlenstoffatomen erfolgen. Heteroalkyl selbst kann als Substituent sowohl über ein Kohlenstoff- als auch über ein Heteroatom mit dem Molekül verknüpft sein.

Exemplarisch werden Vertreter im Folgenden aufgezählt:
Dimethylaminomethyl; Dimethylaminoethyl (1- Dimethylaminoethyl; 2-Dimethylaminoethyl); Dimethylaminopropyl (1-Dimethylaminopropyl, 2-Dimethylaminopropyl, 3-Dimethylaminopropyl); Diethylaminomethyl; Diethylaminoethyl (1-Diethylaminoethyl, 2-Diethylaminoethyl); Diethylaminopropyl (1-Diethylaminopropyl, 2- Diethylaminopropyl, 3-Diethylaminopropyl); Diisopropylaminoethyl (1-Diisopropylaminoethyl, 2-Diisopropylaminoethyl); Bis-2-methoxyethylamino; [2-(Dimethylamino-ethyl)-ethyl-amino]-methyl; 3-[2-(Dimethylamino-ethyl)-ethyl-amino]-propyl; Hydroxymethyl; 2-Hydroxyethyl; 3-Hydroxypropyl; Methoxy; Ethoxy; Propoxy; Methoxymethyl; 2-Methoxyethyl etc.

Halogenalkyl bezieht sich auf Alkylreste, in denen ein oder mehrere Wasserstoffatome durch Halogenatome ersetzt sind. Halogenalkyl umfasst sowohl gesättigte Alkylreste als auch ungesättigte Alkenyl- und Alkinylreste, wie beispielsweise -CF₃, -CHF₂, -CH₂F, -CF₂CF₃,-CHFCF₃, -CH₂CF₃, -CF₂CH₃, -CHFCH₃, -CF₂CF₂CF₃, -CF₂CH₂CH₃, -CF=CF₂, -CCl=CH₂, -CBr=CH₂, -Cl=CH₂, -C≡C-CF₃, -CHFCH₂CH₃ und -CHFCH₂CF₃.

Halogen bezieht sich auf Fluor-, Chlor-, Brom- und/oder lodatome.

Unter Cycloalkyl ist ein mono- oder bizyklischer Ring zu verstehen, wobei das Ringsystem ein gesättigter Ring aber auch ein ungesättigter, nichtaromatischer Ring sein kann, welcher gegebenenfalls auch Doppelbindungen enthalten kann, wie zum Beispiel Cyclopropyl, Cyclopropenyl, Cyclobutyl, Cyclobutenyl, Cyclopentyl, Cyclopentenyl, Cyclohexyl, Cyclohexenyl, Norbornyl und Norbornenyl.

Cycloalkylalkyl umfasst eine nicht-zyklische Alkylgruppe, in der ein an einem Kohlenstoffatom gebundenes Wasserstoffatom, üblicherweise an einem terminalen C-Atom, durch eine Cycloalkylgruppe ersetzt ist.

Aryl bezieht sich auf monozyklische oder bizyklische aromatische Ringsysteme mit 6 - 10 Kohlenstoffatomen wie beispielsweise Phenyl und Naphthyl.

Arylalkyl umfasst eine nicht-zyklische Alkylgruppe, in der ein an einem Kohlenstoffatom gebundenes Wasserstoffatom, üblicherweise an einem terminalen C-Atom, durch eine Arylgruppe ersetzt ist.

Unter Heteroaryl sind mono- oder bizyklische aromatische Ringsysteme zu verstehen, welche anstelle eines oder mehrere Kohlenstoffatome ein oder mehrere, gleich oder verschiedene Heteroatome enthalten, wie z.B. Stickstoff-, Schwefel- oder Sauerstoffatome. Beispielsweise genannt seien Furyl, Thienyl, Pyrrolyl, Oxazolyl, Thiazolyl, Isoxazolyl, Isothiazolyl, Pyrazolyl, Imidazolyl, Triazolyl, Tetrazolyl, Oxadiazolyl, Thiadiazolyl, Pyridyl, Pyrimidyl, Pyridazinyl, Pyrazinyl und Triazinyl. Beispiele für bizyklische Heteroarylreste sind Indolyl, Isoindolyl, BenzoFuryl, Benzothienyl, Benzoxazolyl, Benzothiazolyl, Benzisoxazolyl, Benzisothiazolyl, Benzimidazolyl, Indazolyl, Isoquinolinyl, Quinolinyl, Quinoxalinyl, Cinnolinyl, Phthalazinyl, Quinazolinyl und Benzotriazinyl, Indolizinyl, Oxazolopyridyl, Imidazopyridyl, Naphthyridinyl, Indolinyl, Isochromanyl, Chromanyl, Tetrahydroisochinolinyl, Isoindolinyl, Isobenzotetrahydrofuryl, Isobenzotetrahydrothienyl, Isobenzothienyl, Benzoxazolyl, Pyridopyridyl, Benzotetrahydrofuryl, Benzotetrahydrothienyl, Purinyl, Benzodioxolyl, Triazinyl, Phenoxazinyl, Phenothiazinyl, Pteridinyl, Benzothiazolyl, Imidazopyridyl, Imidazothiazolyl, Dihydrobenzisoxazinyl, Benzisoxazinyl, Benzoxazinyl, Dihydrobenzisothiazinyl, Benzopyranyl, Benzothiopyranyl, Coumarinyl, Isocoumarinyl, Chromonyl, Chromanonyl, Pyridyl-*N*-oxid Tetrahydroquinolinyl, Dihydroquinolinyl, Dihydroquinolinonyl, Dihydroisoquinolinonyl, Dihydrocoumarinyl, Dihydroisocoumarinyl, Isoindolinonyl, Benzodioxanyl, Benzoxazolinonyl, Pyrrolyl-*N-*oxid, Pyrimidinyl-*N*-oxid, Pyridazinyl-*N*-oxid, Pyrazinyl-*N*-oxid, Quinolinyl-*N*-oxid, Indolyl-*N*-oxid, Indolinyl-*N*-oxid, Isoquinolyl-*N*-oxid, Quinazolinyl-*N*-oxid, Quinoxalinyl-*N*-oxid, Phthalazinyl-*N*-oxid, Imidazolyl-*N*-oxid, Isoxazolyl-*N*-oxid, Oxazolyl-*N*-oxid, Thiazolyl-*N*-oxid, Indolizinyl-*N*-oxid, Indazolyl-*N*-oxid, Benzothiazolyl-*N*-oxid, Benzimidazolyl-*N*-oxid, Pyrrolyl-*N*-oxid, Oxadiazolyl-*N*-oxid, Thiadiazolyl-*N*-oxid, Triazolyl-*N*-oxid, Tetrazolyl-*N*-oxid, Benzothiopyranyl-*S*-oxid und Benzothiopyranyl-*S,S-*dioxid.

Heteroarylalkyl umfasst eine nicht-zyklische Alkylgruppe, in der ein an einem Kohlenstoffatom gebundenes Wasserstoffatom, üblicherweise an einem terminalen C-Atom, durch eine Heteroarylgruppe ersetzt ist.

Heterocycloalkyl bezieht sich auf 3 - 12 Kohlenstoffatome umfassende gesättigte oder ungesättigte, nicht aromatische mono-, bizyklische oder überbrückte bizyklische Ringe, welche anstelle eines oder mehrere Kohlenstoffatome Heteroatome, wie Stickstoff, Sauerstoff oder Schwefel, tragen. Beispiele für solche Heterocyloalkylreste sind Tetrahydrofuryl, Pyrrolidinyl, Pyrrolinyl, Imidazolidinyl, Imidazolinyl, Pyrazolidinyl, Pyrazolinyl, Piperidinyl, Piperazinyl, Indolinyl, Isoindolinyl, Morpholinyl, Thiomorpholinyl, Homomorpholinyl, Homopiperidinyl, Homopiperazinyl, Homothiomorpholinyl, Thiomorpholinyl-*S*-oxid, Thiomorpholinyl-*S,S*-dioxid, Tetrahydropyranyl, Tetrahydrothienyl, Homothiomorpholinyl-*S,S*-Dioxid, Oxazolidinonyl, Dihydropyrazolyl, Dihydropyrrolyl, Dihydropyrazinyl, Dihydropyridyl, Dihydropyrimidinyl, Dihydrofuryl, Dihydropyranyl, Tetrahydrothienyl-S-oxid, Tetrahydrothienyl-*S,S*-dioxid, Homothiomorpholinyl-*S*-oxid, 2-Oxa-5-azabicyclo[2.2.1]heptan, 8-Oxa-3-aza-bicyclo[3.2.1]octan, 3,8-Diaza-bicyclo[3.2.1]octan, 2,5-Diaza-bicyclo[2.2.1]heptan, 3,8-Diaza-bicyclo[3.2.1]octan, 3,9-Diazabicyclo[4.2.1]nonan und 2,6-Diaza-bicyclo[3.2.2]nonan.

Heterocycloalkylalkyl bezieht sich auf eine nicht-zyklische Alkylgruppe, in der ein an einem Kohlenstoffatom gebundenes Wasserstoffatom, üblicherweise an einem terminalen C-Atom, durch eine Heterocycloalkylgruppe ersetzt ist.

Die nachfolgenden Beispiele illustrieren die vorliegende Erfindung ohne sie jedoch in ihrem Umfang einzuschränken.

### Synthese der Reagenzien

Folgende Verbindungen sind bereits in den Anmeldungen WO2006/040281 beziehungsweise WO2006/040279 beschrieben.

| # | Struktur | # | Struktur |
|---|---|---|---|
| H-1) | 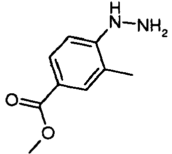 | Z-2) | 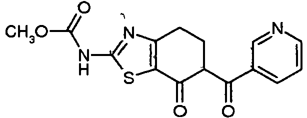 |
| Z-1) | 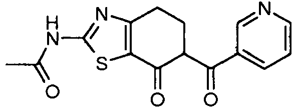 | Z-3) | 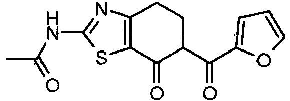 |

### H-2) 3-Brom-4-hydrazino-benzoesäuremethylester

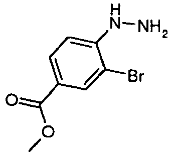

Methyl-4-amino-3-brombenzoat (32 g, 139 mmol) wird mit 250 mL konz. Salzsäure versetzt und auf -10 °C abgekühlt. Eine Lösung von Natriumnitrit (10.2 g, 146 mmol) in 120 mL Wasser wird so zugetropft, dass die Temperatur -5 °C nicht übersteigt. Nach 40 min Rühren bei -10 °C wird zu der Suspension eine Lösung von Zinn(II)chlorid Dihydrat (128 g, 556 mmol) in 130 mL konz. Salzsäure getropft, wobei die Reaktionstemperatur -5 °C nicht übersteigt. Die dickflüssige Suspension wird 1.5 h bei RT gerührt, bevor sie mit NaOH (12 N) auf pH 10 eingestellt wird. Die Reaktionsmischung wird mit je 500 mL Dichlormethan und Wasser versetzt und nach 30 min Rühren filtriert. Der Filterkuchen wird mit 300 mL Dichlormethan und 100 mL Wasser versetzt und 30 min unter Rückfluss erhitzt. Nach Filtration wird das Filtrat mit Chloroform extrahiert. Die vereinigten organischen Phasen werden über Magnesiumsulfat getrocknet, filtriert und zur Trockene eingeengt. Ausbeute: 34 g.

### H-3) 2,5-Difluor-4-hydrazino-benzoesäure

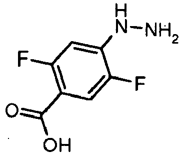

Ausgehend von 4-Nitro-2,5-difluorobenzoesäure (2.5 g, 12.3 mmol) wird durch katalytische Hydrierung mit 10%-Palladium auf Aktivkohle (300 mg) in 100 mL Methanol bei 4 bar Wasserstoffdruck nach Filtration und Evaporation des Lösungsmittels die entsprechende Anilinverbindung 4-Amino-2,5-difluorbenzoesäure erhalten. Ausbeute: 2.1 g.

Analog zur Darstellung von H-2 wird ausgehend von 4-Amino-2,5-difluorbenzoesäure (1.1 g, 6.6 mmol), Natriumnitrit (0.56 g, 7.8 mmol), Zinn(II)chlorid Dihydrat (4.4 g, 20 mmol) in insgesamt 8 mL konz. Salzsäure und 12 mL Eiswasser das gewünschte Produkt H-3 erhalten. Ausbeute: 758 mg.

### H-4) 2,5-Difluor-4-hydrazino-benzoesäuremethylester

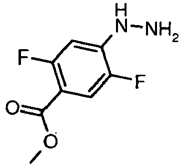

Thionylchlorid (1.5 mL) wird langsam bei 0 °C in 8 mL Methanol getropft. Diese Mischung wird mit einer Lösung von 4-Amino-2,5-difluorbenzoesäure (0.95 g, 5.5 mmol) in 20 mL Methanol über einen Zeitraum von 10 min versetzt, 30 min bei RT und 3 h bei 50 °C gerührt. Nach Evaporation des Lösungsmittels wird der Rückstand in Ethylacetat aufgenommen, und diese Lösung wird 3x mit gesättigter Natriumhydrogencarbonatlösung gewaschen, über Magnesiumsulfat getrocknet und eingeengt. Ausbeute: 1 g Methyl-4-amino-2,5-difluorbenzoat

Analog zur Darstellung von H-2 wird ausgehend von Methyl-4-amino-2,5-difluorbenzoat (1 g, 5.4 mmol), Natriumnitrit (0.5 g, 7.5 mmol), Zinn(II)chlorid Dihydrat (3.6 g, 16 mmol) in insgesamt 8 mL konz. Salzsäure und 10 mL Eiswasser das gewünschte Produkt H-4 erhalten. Ausbeute: 246 mg.

### H-5) 2-Brom-4-isopropylphenylhydrazin

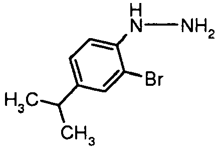

Analog zur Darstellung von H-2 wird ausgehend von 2-Brom-4-isopropylanilin (1 g, 4.5 mmol), Natriumnitrit (0.38 g, 5.4 mmol), Zinn(II)chlorid Dihydrat (2.6 g, 11.3 mmol) in insgesamt 55 mL Salzsäure (w = 0.37) und 20 mL Wasser das gewünschte Produkt H-5 erhalten. Ausbeute: 0.8 g.

### H-6) 2-Fluor-4-(2-morpholin-4-yl-ethoxy)-phenylhydrazin

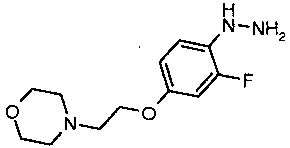

Zu einer Lösung von 3-Fluor-4-Nitrophenol (1.6 g, 10 mmol) in 10 mL DMF werden nacheinander Kaliumcarbonat (2.7 g, 20 mmol) und *N*-(2-Chlorethyl)morpholin Hydrochlorid (1.9 g, 10 mmol) gegeben. Die Reaktionsmischung wird 4 h bei 80 °C gerührt, mit Wasser versetzt und mit Ethylacetat extrahiert. Die vereinigten organischen Phasen werden nacheinander mit 1 N NaOH und gesättigter Natriumchloridlösung gewaschen, über Magnesiumsulfat getrocknet und im Vakuum eingeengt. Das Rohprodukt wird an Kieselgel mit 0 - 60% Ethylacetat in Cyclohexan chromatographisch gereinigt. Das so erhaltene Produkt 4-[2-(3-Fluor-4-nitro-phenoxy)-ethyl]-morpholin (2 g, 7 mmol) wird einer katalytischen Hydrierung mit 10%-Palladium auf Aktivkohle (0.1 g) in 100 mL Methanol in Wasserstoffatmosphäre unterzogen, wobei das gebildete 2-Fluor-4-(2-morpholin-4-yl-ethoxy)anilin nach Filtration und Evaporation des Lösungsmittels als Feststoff anfällt. Ausbeute: 1.7 g.

Zu einer Lösung von 2-Fluor-4-(2-morpholin-4-yl-ethoxy)anilin (1.7 g, 4.5 mmol) in 20 mL konz. Salzsäure wird bei -15 °C langsam eine Lösung von Natriumnitrit (0.38 g, 5.4 mmol) in 20 mL Wasser getropft. Die entstandene Suspension wird 4 h bei -10 °C gerührt, bevor eine Lösung von Zinn(II)chlorid Dihydrat (2.6 g, 11.3 mmol) in 20 mL konz. Salzsäure zugetropft wird. Nach 14 h Rühren bei RT wird die Reaktionsmischung mit 10 N NaOH auf pH 10 gestellt und mit Dichlormethan versetzt. Nach Filtration über Celite® wird das Filtrat mit Dichlormethan extrahiert. Die vereinigten organischen Phasen werden über Magnesiumsulfat getrocknet und im Vakuum eingeengt. Ausbeute: 1.5 g.

### H-7) 5-Cyano-2,4-difluorphenylhydrazin

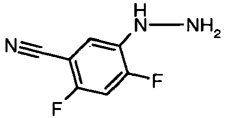

2,4-Difluor-5-nitrobenzonitril (3 g, 16 mmol) wird zunächst einer katalytischen Hydrierung mit 5%-Palladium auf Aktivkohle (0.25 g) in 250 mL Methanol unterzogen (14 h bei 4 bar Wasserstoffdruck), wobei das gebildete 5-Amino-2,4-difluorbenzonitril nach Filtration und Evaporation des Lösungsmittels als Feststoff anfällt. Ausbeute: 2.3 g. Analog zur Darstellung von H-2 wird ausgehend von 5-Amino-2,4-difluorbenzonitril (1.4 g, 9 mmol), Natriumnitrit (0.8 g, 12 mmol), Zinn(II)chlorid Dihydrat (7.2 g, 32 mmol) in insgesamt 40 mL Salzsäure (w = 0.32) und 13 mL Wasser das gewünschte Produkt H-7 erhalten. Ausbeute: 0.55 g.

### H-8) Methyl-3-methoxy-4-hydrazinobenzoat

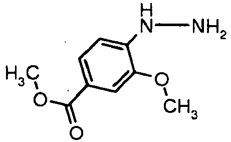

Analog zur Darstellung von H-2 wird ausgehend von Methyl-4-amino-3-methoxybenzoat (5.2 g, 28.4 mmol), Natriumnitrit (2.2 g, 31 mmol), Zinn(II)chlorid Dihydrat (27.6 g, 122 mmol) in insgesamt 50 mL konz. Salzsäure und 25 mL Wasser das gewünschte Produkt H-8 erhalten. Ausbeute: 4.5 g.

Alle weiteren zur Synthese der Beispiele verwendeten Reagenzien sind kommerziell erhältlich oder deren Herstellung ist literaturbekannt.

### Synthese der Beispiele

### I-1) 4-(7-Acetylamino-3-pyridin-3-yl-4,5-dihydro-pyrazolo[4,3-g]benzthiazol-1-yl)-3-brom-benzoesäuremethylester

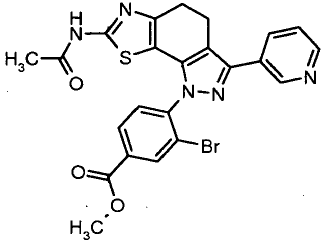

Eine Suspension von Z-1 (10 g, w=0.9, 29 mmol) und H-2 (8 g, 33 mmol) in 30 mL Eisessig wird 15 h bei RT gerührt. Anschließend wird die Reaktionsmischung eingeengt mit 60 mL Ethanol versetzt und 30 min bei 35 °C gerührt. Die Suspension wird langsam abgekühlt, filtriert, der erhaltene Feststoff mit Ethanol (3x 15 mL) gewaschen und im Vakuum getrocknet. Ausbeute: 11.8 g.

### I-2) 3-Brom-4-(7-methoxycarbonylamino-3-pyridin-3-yl-4,5-dihydro-pyrazolo[4,3-g]benzthiazol-1-yl)-benzoesäuremethylester

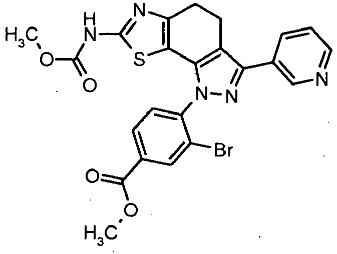

Analog zur Darstellung von I-1 wird ausgehend von Z-2 (1.6 g, 4.7 mmol), H-2 (1.16 g, 4.8 mmol) und 15 mL Eisessig die gewünschte Verbindung 1-2 als Feststoff erhalten. Ausbeute: 2.2 g.

### I-3) 3-Fluor-4-(7-methoxycarbonylamino-3-pyridin-3-yl-4,5-dihydro-pyrazolo[4,3-g]benzthiazol-1-yl)- benzoesäuremethylester

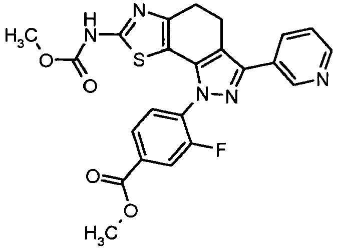

Analog zur Darstellung von I-1 wird ausgehend von Z-2 (1 g, 3.0 mmol), H-1 (1.1 g, 6 mmol) und 13 mL Eisessig die gewünschte Verbindung 1-3 als Feststoff erhalten. Ausbeute: 0.75 g.

### I-4) N-[1-(2-Fluor-4-nitro-phenyl)-3-pyridin-3-yl-4,5-dihydro-1H-pyrazolo[4,3-g]benzthiazol-7-yl]-acetamid

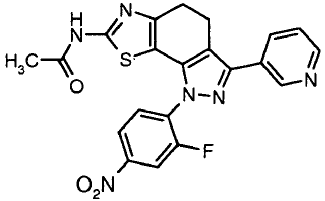

Die Synthese ist in PCT/EP05055021 beschrieben.

### I-5) 2,5-Difluor-4-(7-methoxycarbonylamino-3-pyridin-3-yl-4,5-dihydro-pyrazolo[4,3-g]benzthiazol-1-yl)-benzoesäure

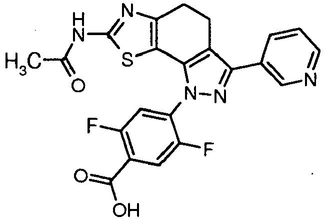

Analog zur Darstellung von I-1 wird ausgehend von Z-1 (1 g, 3.3 mmol), H-3 (0.75 mg, 4 mmol) und 20 mL Eisessig die gewünschte Verbindung 1-5 als Feststoff erhalten. Ausbeute: 0.9 g.

### I-6) N-[1-(4-Nitro-2-trifluormethyl-phenyl)-3-pyridin-3-yl-4,5-dihydro-1H-pyrazolo[4,3-g]benzthiazol-7-yl]-acetamid

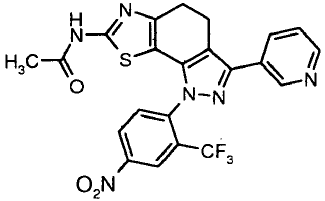

Eine Suspension von Z-1 (1.9 g, 6 mmol) und 4-Nitro-2-trifluormethyl-phenylhydrazin (1.5 g, 6.6 mmol) in 37 mL Eisessig wird 1 h bei 60 °C gerührt. Anschließend wird die Reaktionsmischung auf Eiswasser gegeben und mit Ethylacetat extrahiert. Die vereinigten organischen Phasen werden mit gesättigter Natriumchloridlösung gewaschen, über Natriumsulfat getrocknet, filtriert und im Vakuum eingeengt. Das Rohprodukt wird in einem Gemisch aus 8 mL Acetonitril, 8 mL DMF und 5 mL DMSO gelöst und mittels RP-Chromatographie gereinigt (Gradient: 10 % Acetonitril/Wasser → 98 %; 80 min) Acetonitril Wasser in 80 min. Das isolierte Produkt wird anschließend aus Dichlormethan /Methanol (5:1) umkristallisiert. Ausbeute: 340 mg.

### I-7) N-[1-(4-Nitro-2-trifluormethyl-phenyl)-3-furan-2-yl-4,5-dihydro-1H-pyrazolo[4,3-g]benzthiazol-7-yl]-acetamid

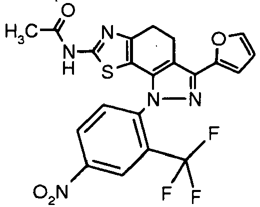

Analog zur Darstellung von 1-6 wird ausgehend von Z-3 (5 g, 16.4 mmol), 4-Nitro-2-trifluormethyl-phenylhydrazin (4 g, 18 mmol) in 100 mL Eisessig das gewünschte Produkt nach Chromatographie an Kieselgel (Cyclohexan / Ethylacetat 60:40 → 70:30) erhalten. Ausbeute: 1.3 g.

### I-8) 3-Methoxy-4-(7-methoxycarbonylamino-3-pyridin-3-yl-4,5-dihydro-pyrazolo[4,3-g]benzthiazol-1-yl)-benzoesäuremethylester

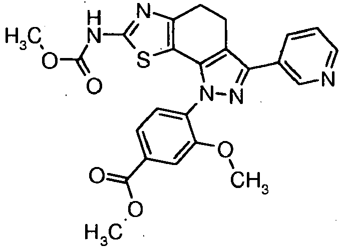

Analog zur Darstellung von I-1 wird ausgehend von Z-2 (2.57 g, 7.8 mmol), H-8 (3 g, 15.5 mmol) und 42 mL Eisessig die gewünschte Verbindung I-8 als Feststoff erhalten. Ausbeute: 0.96 g.

### II-1) 4-(7-Acetylamino-3-pyridin-3-yl-4,5-dihydro-pyrazolo[4,3-g]benzthiazol-1-yl)-3-brom-benzoesäure

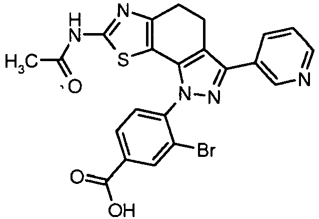

Zu einer Suspension von I-1 (2.2 g, 4.1 mmol) in Dioxan wird unter Rühren eine Lösung Lithiumhydroxid (0.7 g, 29 mmol in 4.3 mL Wasser) getropft. Nach Aufklaren der Lösung wird die Reaktionsmischung weitere 30 min bei RT gerührt, mit 20 mL Wasser verdünnt und mit 1 N Salzsäure auf pH 5 gestellt. Der entstehende Niederschlag wird filtriert, mit Wasser gewaschen und getrocknet. Das gewünschte Produkt wird ohne weitere Reinigung verwendet. Ausbeute: 1.9 g.

Analog der Darstellung von II-1 werden folgende Carbonsäuren erhalten.

| # | Edukt | Struktur | # | Edukt | Struktur |
|---|---|---|---|---|---|
| II-2 | I-2 | 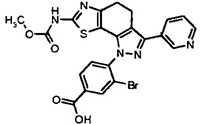 | II-3 | I-3 | 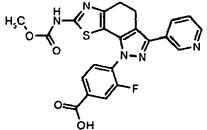 |
| II-4 | I-8 | 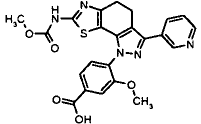 | | | |

### III-1) N-[1-(4-Amino-2-fluorphenyl)-3-pyridin-3-yl-4,5-dihydro-1H-pyrazolo[4,3-g]benzthiazol-7-yl]-acetamid

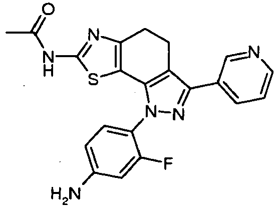

Die Synthese ist in PCT/EP05055021 beschrieben.

### III-2) 1-(2-Fluor-4-nitro-phenyl)-3-pyridin-3-yl-4,5-dihydro-1H-pyrazolo[4,3-g]benzthiazol-7-ylamin

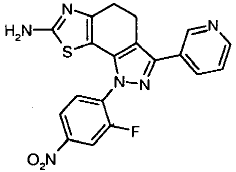

Eine Lösung von 1-4 (0.5 g, 1.1 mmol) in 6 N Salzsäure (10 mL) wird 5 h bei 70 °C und anschließend 14 h bei 60 °C gerührt. Nach Abkühlen wird die Reaktionsmischung mit 1 N NaOH leicht basisch gestellt und mit Ethylacetat extrahiert. Die vereinigten organischen Phasen werden über Magnesiumsulfat getrocknet, filtriert und im Vakuum eingeengt. Das Rohprodukt wird ohne weitere Reinigung verwendet.

### III-3) 1-(2-Fluor-4-nitro-phenyl)-3-pyridin-3-yl-4,5-dihydro-1H-pyrazolo[4,3-g]benzthiazol-7-yl-carbamidsäuremethylester

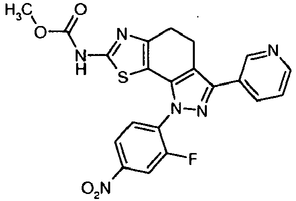

Eine Lösung von III-2 (460 mg, 1.1 mmol) in 10 mL Pyridin wird auf -20 °C gekühlt und portionsweise mit Methylchloroformiat (0.1 mL, 1.3 mmol) versetzt. Nach 14 h Rühren bei RT wird der gebildete Niederschlag filtriert, in Acetonitril aufgenommen und 30 min im Ultraschall behandelt. Der nicht gelöste Feststoff wird filtriert und getrocknet. Ausbeute: 270 mg.

### III-4) 1-(4-Amino-2-fluorphenyl)-3-pyridin-3-yl-4,5-dihydro-1H-pyrazolo{4,3-g]benzthiazol-7-yl-carbamidsäuremethylester

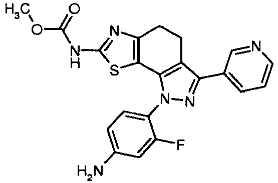

Eine Lösung von III-3 (265 mg, 0.6 mmol) in 20 mL THF und 20 mL DMF wird mit 10%-Platin auf Aktivkohle (36 mg) sowie Vanadylacetylacetonat (37 mg, 0.14 mmol) versetzt und 14 h bei RT unter 4 bar Wasserstoffatmosphäre gerührt und anschließend filtriert. Das Filtrat wird im Vakuum eingeengt. Ausbeute: 242 mg

### III-5) N-1-(4-Amino-2-trifluormethylphenyl)-3-pyridin-3-yl-4,5-dihydro-1H-pyrazolo[4,3-g] benzthiazol-7-yl-acetamid

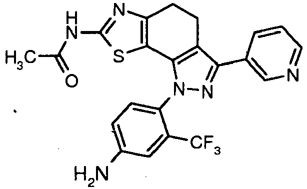

Analog zur Darstellung von III-4 wird ausgehend von 1-6 (333mg, 0.67 mmol), 5%-Platin auf Aktivkohle (75 mg) und Vanadylacetylacetonat (65 mg, 0.24 mmol) in 50 mL Methanol und 10 mL DMF das gewünschte Produkt III-5 erhalten. Ausbeute: 313 mg.

### III-6) N-1-(4-Amino-2-trifluormethylphenyl)-3-furan-2-yl-4,5-dihydro-1H-pyrazolo[4,3-g] benzthiazol-7-yl-acetamid

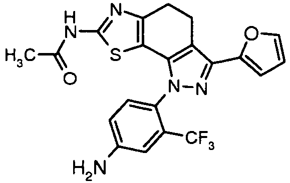

Analog zur Darstellung von III-4 wird ausgehend von 1-7 (333mg, 0.67 mmol), 5%-Platin auf Aktivkohle (75 mg) und Vanadylacetylacetonat (65 mg, 0.24 mmol) in 50 mL Methanol und 10 mL DMF das gewünschte Produkt III-6 erhalten. Ausbeute: 313 mg.

### IV-1) N-[4-(7-Acetylamino-3-pyridin-3-yl-4,5-dihydro-pyrazolo[4,3-g]benzthiazol-1-yl)-3-fluorphenyl]-2-chloracetamid

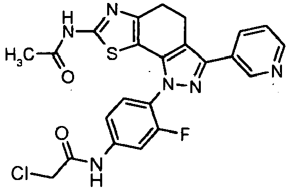

Eine Suspension von III-1 (928 mg, 1.8 mmol) in 20 mL NMP wird mit 0.5 mL Diisopropylethylamin versetzt und 5 min bei RT gerührt. Nach Zugabe von 0.54 mL Chloracetylchlorid wird 1 h bei RT gerührt, anschließend mit Wasser versetzt und mit Dichlormethan extrahiert. Die vereinigten organischen Phasen werden über Magnesiumsulfat getrocknet, filtriert und eingeengt. Nach Reinigung mittels RP-Chromatographie (Gradient: 5% Acetonitril/ Wasser → 98 % Acetonitril / Wasser; 35 min) werden die Produkt enthaltenden Fraktionen lyophilisiert. Ausbeute: 364 mg.

### IV-2 N-[4-(7-Acetylamino-3-pyridin-3-yl-4,5-dihydro-pyrazolo[4,3-g]benzthiazol-1-yl)-3-fluorphenyl]-3-chlorpropionamid

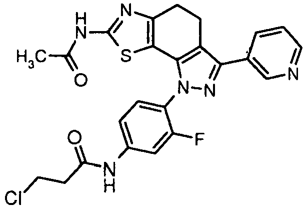

Eine Suspension von III-1 (1.5 g, w = 0.75, 2.7 mmol) in 12 mL NMP wird mit 0.5 mL Diisopropylethylamin versetzt und 5 min bei RT gerührt. Nach Zugabe von 0.44 mL 3-Chlorpropionylchlorid wird 1 h bei RT gerührt, anschließend mit Wasser versetzt und mit Dichlormethan extrahiert. Die vereinigten organischen Phasen werden über Magnesiumsulfat getrocknet, filtriert und eingeengt. Nach Reinigung mittels RP-Chromatographie (Gradient: 5% Acetonitril / Wasser → 98 % Acetonitril / Wasser; 35 min) werden die Produkt enthaltenen Fraktionen lyophilisiert. Ausbeute: 624 mg.

### IV-3 [4-(7-Acetylamino-3-pyridin-3-yl-4,5-dihydro-pyrazolo[4,3-g]benzthiazol-1-yl)-3-fluorphenyl]-carbamidsäure-2-chlorethylester

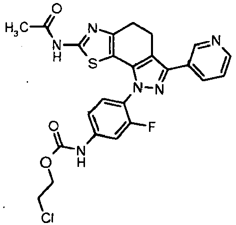

Eine Suspension von III-1 (60 mg, 0.14 mmol) in 3 mL NMP wird mit 80 µL Diisopropylethylamin versetzt und 5 min bei RT gerührt. Nach Zugabe von 65 µL 2-Chlorethylchloroformiat wird 1 h bei RT gerührt, anschließend mit DMF versetzt. Nach Reinigung mittels RP-Chromatographie (Gradient: 5% Acetonitril / Wasser → 70 % Acetonitril / Wasser; 40 min) werden die Produkt enthaltenen Fraktionen lyophilisiert. Ausbeute: 12 mg.

### IV-4 N-[4-(7-Acetylamino-3-pyridin-3-yl-4,5-dihydropyrazolo[4,3-g]benzthiazol-1-yl)-3-fluorphenyl]-2-chlorpropionamid

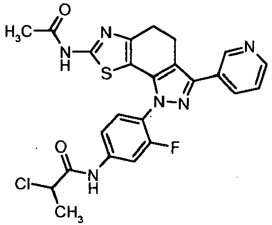

Eine Suspension von III-1 (1.1 g, 2.6 mmol) in 10 mL NMP wird mit 0.9 mL Diisopropylethylamin versetzt und 5 min bei RT gerührt. Nach Zugabe von 0.5 mL 2-Chlorpropionylchlorid wird 1 h bei RT gerührt, anschließend mit DMF versetzt. Nach Reinigung mittels RP-Chromatographie (Gradient: 5% Acetonitril / Wasser → 70 % Acetonitril / Wasser; 40 min) werden die Produkt enthaltenen Fraktionen lyophilisiert. Ausbeute: 382 mg.

### Analytische Methoden

### Methode AM1:

HPLC: Agilent 1100 Series; MS: 1100 Series LC/MSD (API-ES (+/- 3000V, Quadrupol, G1946D); Mode: Scan pos 100-1000, neg 100-1000
   - Säule:: Waters; Part No.186000594; XTerra MS C18 2.5µm; 2.1x50mm column
   - Lösungsmittel:: A: H₂O entsalzt mit 0.1 % Ameisensäurezusatz
   B: Acetonitril HPLC grade mit 0.1 % Ameisensäurezusatz
   - Detektion:: peakwide >0.1 min (2 s); 190 - 450 nm
   UV 254 nm (bandwide 8, reference off)
   UV 230 nm (bandwide 8, reference off)
Injektion: 1 µL standard injection
Fluss: 0.6 mL/min
Säulentemperatur: 35 °C
   - Pumpengradient:: 0.0 - 0.5 min 5% B
   0.5 - 1.5 min 5% → 50% B
   1.5 - 4.0 min 50% → 95% B
   4.0 - 6.0 min 95% B
   6.0 - 6.5 min 95% → 5% B
   1.5 min post run 5% B

### Methode AM2

HPLC: Agilent Series 1100 (G1379A/G1310A umgerüstet auf G1311A/G1313A/G1316A/G1948D/G1315B/G1946D) Mode: Scan pos 100-1000, neg 100-1000
   - Säule:: Agilent Zorbax SB-C8, 2.1x50 mm, 3.5 µm
   - Lösungsmittel:: A: H₂O entsalzt mit 0.1%-Ameisensäurezusatz
   B: Acetonitril HPLC grade mit 0.1 % Ameisensäurezusatz
   - Detektion:: peakwide >0.1 min (2 s); 190 - 450nm
   UV 254 nm (bandwide 8, reference off)
   UV 230 nm (bandwide 8, reference off)
Injektion: 2.5 µL standard injection
Fluss: 0. 6 mL/min
Säulentemperatur: 35 °C
   - Pumpengradient:: 0 - 3.0 min 10% -> 90% B
   3.0 - 4.0 min 90% B
   4.0 - 5.0 min 90% → 10% B

### Methode AM3

HPLC: Agilent Series 1100 (G1312A/G1315A/G1316A/G1367A) Agilent MSD SL ESI Mode: Scan pos 150-750
Säule: Agilent Zorbax SB-C8, 2.1x50 mm, 3.5 µm
   - Lösungsmittel:: A: H₂O entsalzt mit 0.1% Ameisensäurezusatz
   B: Acetonitril HPLC grade mit 0.1 % Ameisensäurezusatz
   - Detektion:: peakwidth >0.01 min (0.2 s); 190 - 450nm
   UV 254 nm (bandwide 16, reference off)

   UV 230 nm (bandwide 8, reference off)

   UV 214 nm (bandwide 8, reference off)
   - Injektion:: 3.0 µL overlap injection
   - Fluss:: 1.1 mL/min
   - Säulentemperatur:: 45 °C
   - Pumpengradient:: 0 - 1.75 min 15% -> 95% B
   1.75 - 1.90 min 95% B
   1.90 - 1.92 min 950% -> 15% B

### Methode AM4

| | |
|---|---|
| HPLC: | Agilent 1100 Series |
| MS: | Agilent LC/MSD SL (LCMS1: 1100 series LC/MSD) |
| Säule: | Waters, Xterra MS C18, 2.5µm, 2.1x30 mm, Part.No.186000592 |
| Lösungsmittel | A: H₂O entsalzt mit 0.1% Ameisensäurezusatz |
| | B: Acetonitril HPLC grade mit 0.1% Ameisensäurezusatz |
| Detektion: | MS: Positive and negative |
| | Mass range: 120 - 900 m/z |
| | Fragmentor: 120 |
| Gain EMV: | 1 |
| Threshold: | 150 |
| Stepsize: | 0.25 |
| | UV: 254 nm |
| Bandwide: | 1 (LCMS1: 2) |
| Reference: | off |

### Spectrum:

| | | | |
|---|---|---|---|
| | Range: | 250 - 400 nm | |
| | Range step: | 1.00 nm | |
| | Threshold: | 4.00 mAU | |
| | Peakwidth: | < 0.01 min | (LCMS1: >0.05 min) |
| | Slit: | 1 nm | (LCMS1: 2 nm) |
| Injektion: | 5 µL | | |
| Fluß: | 1.10 mL/min | | |
| Säulentemperatur: | 40 °C | | |
| Gradient: | 0.00 min | | 5 % B |
| | 0.00 - 2.50 min | | 5 % -> 95 % B |
| | 2.50 - 2.80 min | | 95 % B |
| | 2.81 - 3.10 min | | 95 % → 5 % B |

### Methode AM5

| | |
|---|---|
| HPLC: | Agilent 1100 Series |
| MS: | Agilent LC/MSD SL (LCMS1: 1100 series LC/MSD) |
| Column: | Phenomenex, Synergi Polar RP 80A, 4 µm, 2x30 mm, Part.No.00A-4336-B0 |
| Solvent: | A: H₂O (Millipore purified purest water) with 0.1 % Ameisensäurezusatz |
| | B: Acetonitril (HPLC grade) |
| Detection: | MS: Positive and negative |
| | Mass range: 120 - 900 m/z |
| | Fragmentor: 120 |
| Gain EMV: | 1 |
| Threshold: | 150 |
| Stepsize: | 0.25 |
| UV: | 254 nm |
| Bandwide: | 1 (LCMS1: 2) |
| Reference: | off |

### Spectrum:

| | |
|---|---|
| Range: | 250 - 400 nm |
| Range step: | 1.00 nm |
| Threshold: | 4.00 mAU |
| Peakwidth: | < 0.01 min (LCMS1: >0.05 min) |
| Slit: | 1 nm (LCMS1: 2 nm) |
| Injection: | Inj. Vol.: 5 µL |
| Inj. mode: | Needle wash |
| Separation: | Flow: 1.10 mL/min |
| Column temp.: | 40 °C |
| Gradient: | 0.00 min 5 % solvent B |
| | 0.00 - 2.50 min 5 % -> 95 % solvent B |
| | 2.50 - 2.80 min 95 % solvent B |
| | 2.81 - 3.10 min 95 % → 5 % solvent B |

### Methode AM6

| | |
|---|---|
| HPLC: | Waters Alliance 2695 |
| Säule: | Waters, Xterra MS C18, 2.5 µm, 4.6x30 mm, Part.No.186000600 |
| Lösungsmittel | A: H₂O entsalzt mit 0.1 % Ameisensäurezusatz |
| | B: Acetonitril HPLC grade mit 0.08 % Ameisensäurezusatz |
| Fluß: | 1 mL/min |
| Säulentemperatur: | 25 °C |
| Gradient: | 0.00 min 5 % B |
| | 0.00 - 3.10 min 5 % -> 98 % B |
| | 3.10 - 4.50 min 98 % B |
| | 4.50 - 5.00 min 98 % → 5 % B |

### Verwendete Abkürzungen

- DC: Dünnschichtchromatographie
- DMF: *N,N*-Dimethylformamid
- DMSO: Dimethylsulfoxid
- h: Stunde
- HATU: *O*-(7-Azabenztriazol-1-yl)-*N,N,N',N'*-tetramethyluroniumhexafluorphosphat
- HPLC: Hochdurchsatzflüssigchromatographie
- konz.: konzentriert
- M: Molar
- min: Minute
- mL: Milliliter
- MS: Massenspektrometrie
- N: Normal
- NaOH: Natriumhydroxid
- NMR: Kernresonanzspektroskopie
- NMP: *N*-Methylpyrrolidinon
- Rf: Retentionsfaktor
- RP: Reversed Phase
- RT: Raumtemperatur
- Rt: Retentionszeit
- Smp: Schmelzpunkt
- *tert*: tertiär
- THF: Tetrahydrofuran
- w: Massengehalt

### Beispiele 1.1 - 1.7

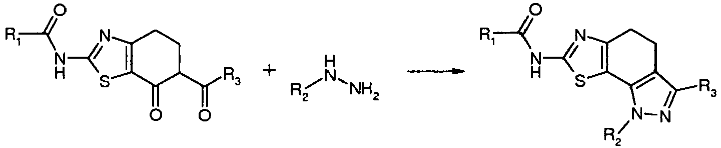

Die Darstellung der Beispiele 1.1 - 1.7 erfolgt analog der Synthese I-1. : Die Beispiele 1.1-1.7 gehören zur vorliegenden Erfindung nicht.

| # | Edukt | Struktur | Masse [M+1]⁺ | HPLC Rt [min] |
|---|---|---|---|---|
| 1.1 | Z-1 | 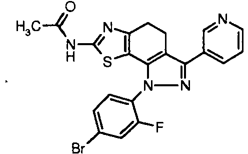 | 484/486 | 1.75 |
| 1.2 | Z-1 H-5 | 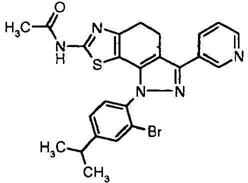 | 508/510 | 1.89 |
| 1.3 | Z-1 H-6 | 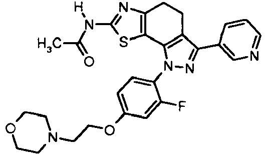 | 535 | 1.31 |
| 1.4 | Z-1 | 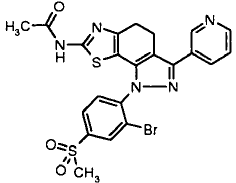 | 544/546 | 1.56 |
| 1.5 | Z-1 | 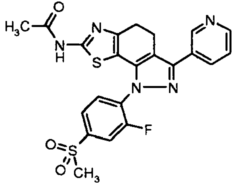 | 484 | 1.53 |
| 1.6 | Z-1 H-4 | 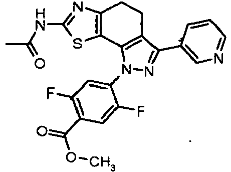 | 482 | 1.75 |
| 1.7 | Z-1 H-7 | 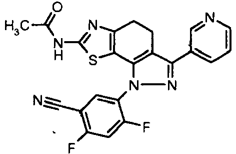 | 449 | 1.64 |

### Umsetzung der Carbonsäuren mit Aminen

### Synthesemethode A

Eine Lösung einer Carbonsäure (0.1 mmol) in 5 mL Dichlormethan oder DMF wird mit *O-*Benztriazol-1-yl-*N*,*N*,*N',N'*-tetramethyluroniumtetrafluorborat (0.15 mmol) und Diisopropylethylamin (0.3 mmol) versetzt und 15 min bei RT gerührt. Anschließend wird das entsprechende Amin (0.1 mmol) hinzugegeben und bei RT bis zum vollständigen Umsatz gerührt. Die Reaktionsmischung wird mit wässriger 5%iger Kaliumcarbonatlösung versetzt und mit Dichlormethan extrahiert. Die vereinigten organischen Phasen werden getrocknet und im Vakuum eingeengt. Der Rückstand wird aus Petrolether kristallisiert oder chromatographisch gereinigt.

### Synthesemethode B

Eine Lösung einer Carbonsäure (0.35 mmol) in 5 mL DMF (oder Dichlormethan, oder THF) wird mit HATU (0.55 mmol) und Diisopropylethylamin (1.8 mmol) versetzt und 15 min bei RT gerührt. Nach Zugabe des entsprechenden Amins (0.39 mmol) wird 15 h bei RT gerührt, mit wässriger 5%iger Kaliumcarbonatlösung versetzt und mit Dichlormethan extrahiert. Die vereinigten organischen Phasen werden getrocknet und im Vakuum eingeengt. Der Rückstand wird chromatographisch gereinigt.

### Synthesemethode C

Die Synthese erfolgt analog Synthesemethode A, allerdings wird Triethylamin statt Diisopropylethylamin verwendet.

### Synthesemethode D

Die Synthese erfolgt analog Synthesemethode B, allerdings wird Triethylamin statt Diisopropylethylamin verwendet.

: Die Beispiele 2.2 - 2.22, 2.24 und 2.31 - 2.34 gehören zur vorliegenden Erfindung nicht.

### Beispiele 2.1 - 2.58

| # | Edukt | Struktur | Masse [M+1]⁺ | HPLC Rt [min] |
|---|---|---|---|---|
| 2.1 | II-2 | 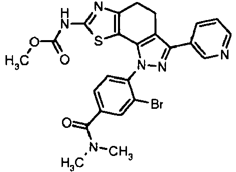 | 553/555 | 1.56 |
| 2.2 | II-1 | 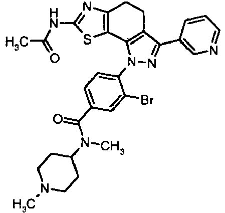 | 620/622 | 1.28 |
| 2.3 | II-1 | 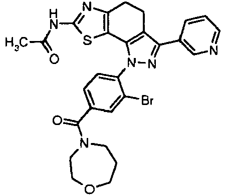 | 503/595 | 1.52 |
| 2.5 | II-1 | 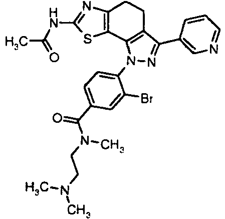 | 594/596 | 0.12 |
| 2.6 | II-1 | 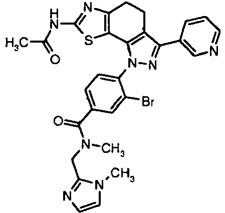 | 617/619 | 0.12 |
| 2.7 | II-1 | 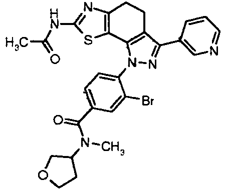 | 593/595 | 1.57 |
| 2.8 | II-1 | 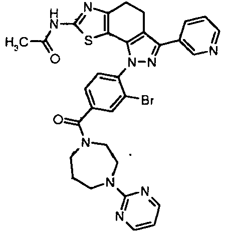 | 670/672 | 1.64 |
| 2.9 | II-1 | 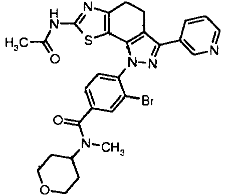 | 607/609 | 1.6 |
| 2.10 | II-1 | 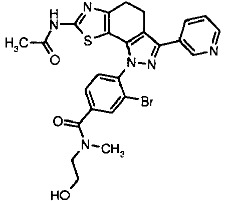 | 567/569 | 0.14 |
| 2.11 | II-1 | 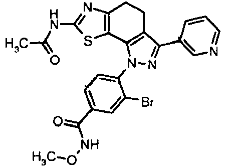 | 539/541 | 1.48 |
| 2.12 | II-1 | 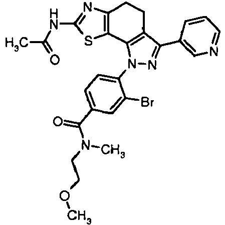 | 581/583 | 1.56 |
| 2.13 | II-1 | 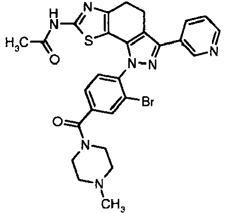 | 592/594 | 0.12 |
| 2.14 | II-1 | 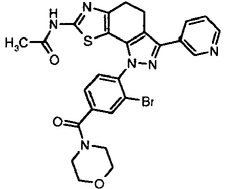 | 579/581 | 1.54 |
| 2.15 | II-1 | 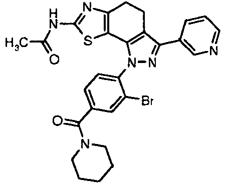 | 577/579 | 1.72 |
| 2.16 | II-1 | 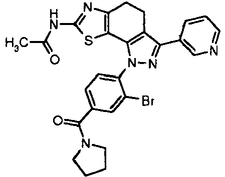 | 563/565 | 1.61 |
| 2.17 | II-1 | 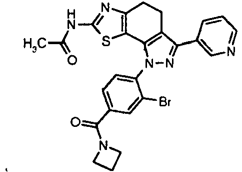 | 549/551 | 1.54 |
| 2.18 | II-1 | 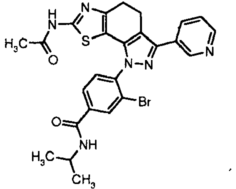 | 551/553 | 1.64 |
| 2.19 | II-1 | 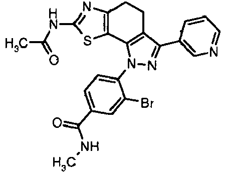 | 523/525 | 1.41 |
| 2.20 | II-1 | 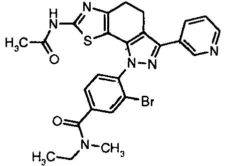 | 551/553 | 1.58 |
| 2.21 | II-1 | 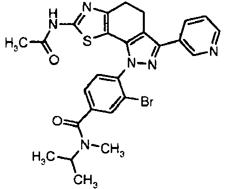 | 565/567 | 1.7 |
| 2.22 | II-1 | 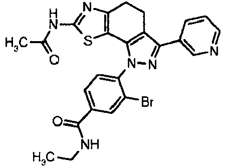 | 537/539 | 1.54 |
| 2.23 | II-1 | 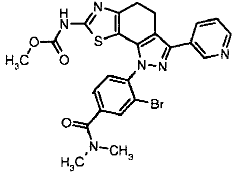 | 537/539 | 1.5 |
| 2.24 | II-1 | 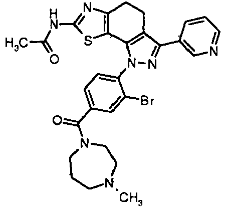 | 606/608 | 1.28 |
| 2.25 | II-3 | 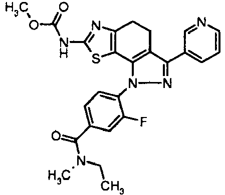 | 507 | 1.62 |
| 2.26 | II-3 | 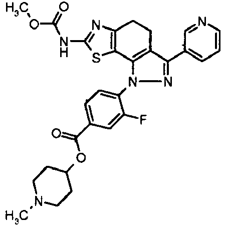 | 563 | 1.4 |
| 2.27 | II-3 | 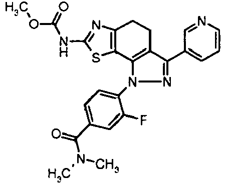 | 593 | 1.56 |
| 2.28 | II-3 | 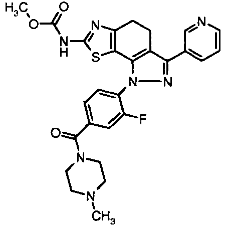 | 548 | 1.31 |
| 2.29 | II-3 | 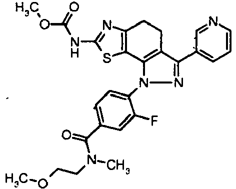 | 537 | 1.6 |
| 2.30 | II-3 | 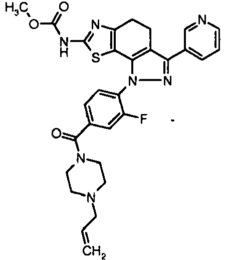 | 574 | 1.41 |
| 2.31 | I-5 | 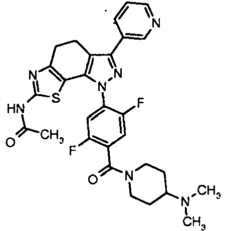 | 578 | 1.34 |
| 2.32 | I-5 | 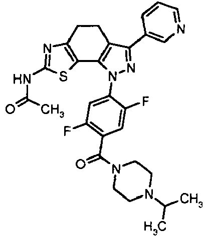 | 578 | 1.39 |
| 2.33 | I-5 | 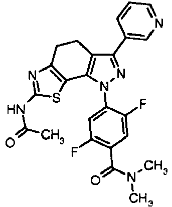 | 495 | 1.57 |
| 2.34 | I-5 | 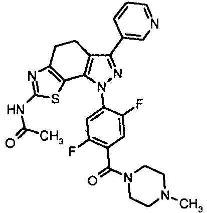 | 550 | 1.32 |
| 2.35 | II-4 | 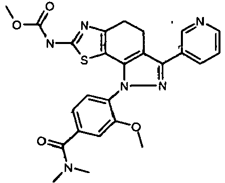 | 505 | 1.47 |
| 2.36 | II-4 | 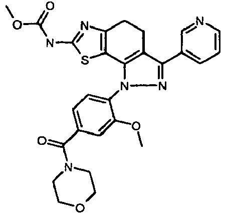 | 547 | 1.49 |
| 2.37 | II-4 | 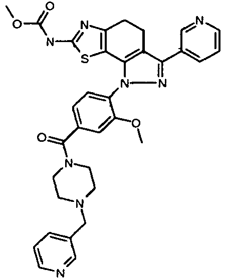 | 637 | |
| 2.38 | II-1 | 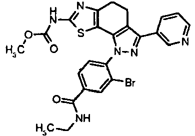 | 553/555 | 1.62 |
| 2.39 | II-1 | 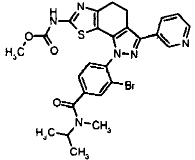 | 581/583 | 1.76 |
| 2.40 | II-1 | 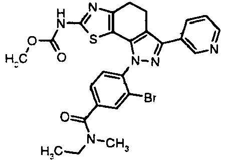 | 567/569 | 1.66 |
| 2.41 | II-1 | 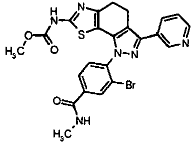 | 539/541 | 1.53 |
| 2.42 | II-1 | 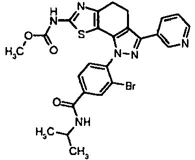 | 567/569 | 1.69 |
| 2.43 | II-1 | 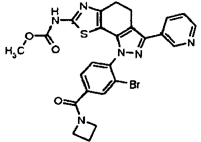 | 565/567 | 1.62 |
| 2.44 | II-1 | 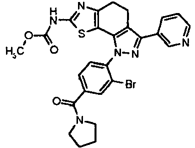 | 579/581 | 1.67 |
| 2.45 | II-1 | 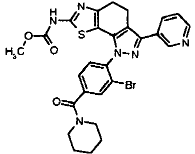 | 583/595 | 1.79 |
| 2.46 | II-1 | 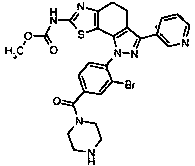 | 594/596 | 1.29 |
| 2.47 | II-1 | 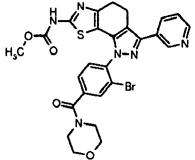 | 595/597 | 1.62 |
| 2.48 | II-1 | 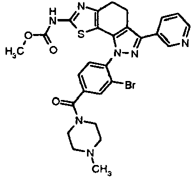 | 608/610 | 1.02 |
| 2.49 | II-1 | 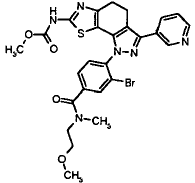 | 597/599 | 1.64 |
| 2.50 | II-1 | 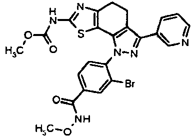 | 555/557 | 1.5 |
| 2.51 | II-1 | 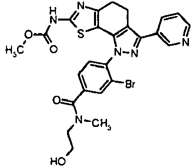 | 583/585 | 1.48 |
| 2.52 | II-1 | 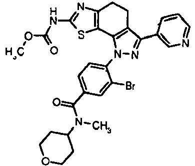 | 623/625 | 1.66 |
| 2.53 | II-1 | 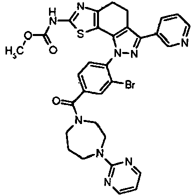 | 686/688 | 1.72 |
| 2.54 | II-1 | 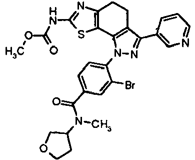 | 609/611 | 1.64 |
| 2.55 | II-1 | 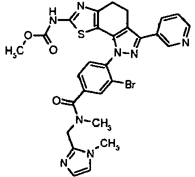 | 633/635 | |
| 2.56 | II-1 | 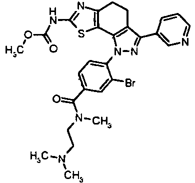 | 610/612 | 1.3 |
| 2.57 | II-1 | 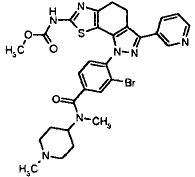 | 636/638 | |
| 2.58 | II-1 | 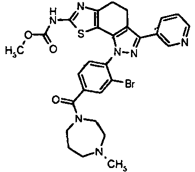 | 622/624 | |

### Umsetzung der hergestellten Amine

### Synthesemethode E - Umsetzung mit Sulfonsäurechloriden

Eine Lösung von 0.2 mmol Amin in 3 mL Pyridin wird mit 0.5 mmol Sulfonsäurechlorid versetzt und 15 h bei RT gerührt. Das Reaktionsgemisch wird eingeengt und der Rückstand chromatographisch gereinigt.

### Synthesemethode F - Umsetzung mit Carbonsäuren

Eine Lösung der Carbonsäure (0.16 mmol) in 1.3 mL DMF wird mit HATU (0.55 mmol) und Diisopropylethylamin (1.8 mmol) versetzt und 1 h bei RT gerührt. Nach Zugabe einer Lösung von 0.1 mmol des entsprechenden Amins in DMF wird weitere 15 h bei RT gerührt. Anschließend wird die Reaktionsmischung filtriert, eingeengt und der Rückstand chromatographisch gereinigt.

### Synthesemethode G - Umsetzung mit Carbonsäurechloriden

Die eingesetzten Carbonsäurechloride sind entweder kommerziell erhältlich oder werden durch Umsetzung der entsprechenden Carbonsäure (0.6 mmol) mit 2 mL Thionylchlorid erhalten.
a) Eine Lösung von 0.2 mmol Amin in 3 mL Pyridin wird mit 0.5 mmol Carbonsäurechlorid versetzt und 15 h bei RT gerührt. Das Reaktionsgemisch wird eingeengt und der Rückstand chromatographisch gereinigt.
b) Die Synthese erfolgt analog Synthesemethode a) jedoch werden statt Pyridin 3 mL DMF und 45 µL Triethylamin verwendet
c) Die Synthese erfolgt analog Synthesemethode a) jedoch werden statt Pyridin 2 mL NMP und 80 µL Diisopropylethylamin verwendet

### Synthesemethode H - Umsetzung_mit Chloroformiaten

Die eingesetzten Chloroformiate sind entweder kommerziell erhältlich oder werden *in situ* durch Umsetzung des entsprechenden Alkohols (1.4 mmol) mit Äquivalenten Phosgen (20% in Toluol; 1.2 mmol) in 1 mL THF hergestellt. Die Reaktionslösung wird direkt ohne weitere Aufarbeitung eingesetzt.

Eine Lösung von 0.14 mmol Amin in 2 mL NMP wird mit 80 µL Diisopropylethylamin versetzt und 5 min gerührt. Anschließend wird das Carbamolychlorid in vier Portionen zu je 10 mg zugegeben und 14 h gerührt. Die Reaktionsmischung wird direkt an RP-Kieselgel chromatographisch gereinigt.

### Beispiele 3.1 - 3.23 Die Beispiele 3.2 - 3.23 gehören zur vorliegenden Erfindung nicht.

| # | Edukt | Struktur | Masse [M+1]⁺ | HPLC Rt [min] |
|---|---|---|---|---|
| 3.1 | III-4 | 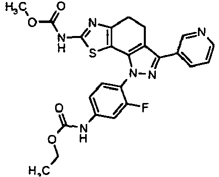 | 509 | 1.71 |
| 3.2 | III-1 | 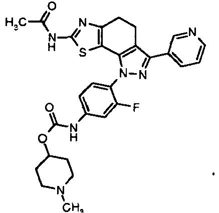 | 562 | 1.39 |
| 3.3 | III-1 | 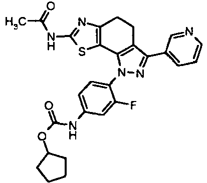 | 533 | 1.88 |
| 3.4 | III-1 | 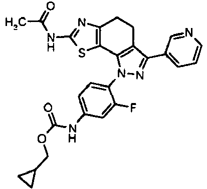 | 519 | 1.78 |
| 3.5 | 111-5 | 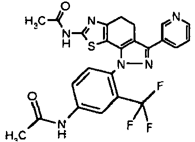 | 513 | 1.52 |
| 3.6 | III-1 | 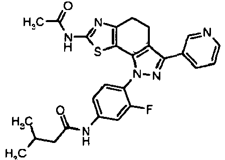 | 505 | 1.75 |
| 3.7 | III-1 | 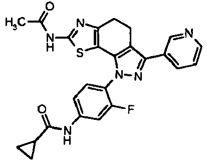 | 489 | 1.61 |
| 3.8 | III-1 | 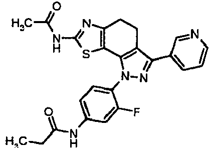 | 477 | 1.55 |
| 3.9 | III-1 | 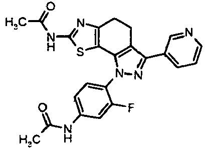 | 463 | 1.46 |
| 3.10 | III-1 | 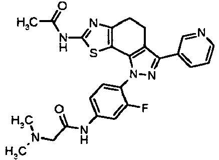 | 506 | 1.3 |
| 3.11 | III-1 | 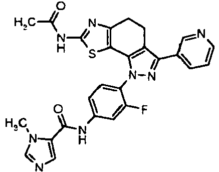 | 529 | 1.43 |
| 3.12 | III-1 | 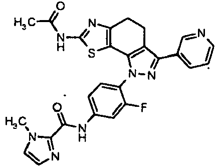 | 529 | 1.66 |
| 3.13 | III-1 | 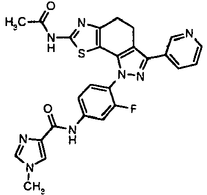 | 529 | 1.44 |
| 3.14 | III-1 | 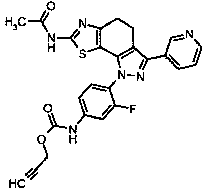 | 503 | 1.66 |
| 3.15 | III-1 | 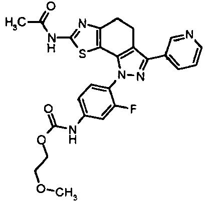 | 523 | 1.6 |
| 3.16 | III-1 | 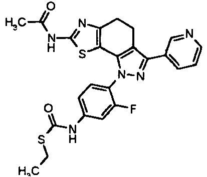 | 509 | 1.78 |
| 3.17 | III-1 | 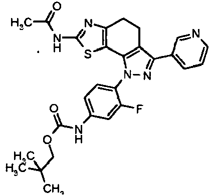 | 535 | 1.96 |
| 3.18 | III-1 | 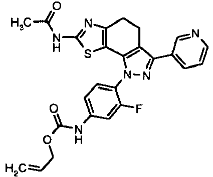 | 505 | 1.73 |
| 3.19 | III-1 | 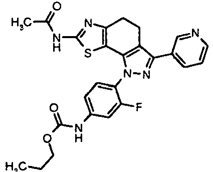 | 507 | 1.77 |
| 3.20 | III-1 | 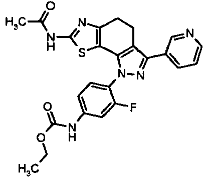 | 493 | 1.66 |
| 3.21 | III-6 | 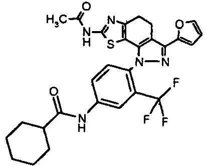 | 570 | 2.33 |
| 3.22 | III-1 | 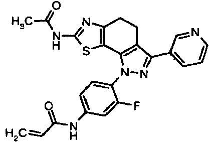 | 475 | 1.56 |
| 3.23 | III-1 | 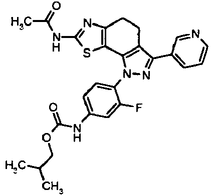 | 521 | 1.88 |

Die Beispiele 4.1- 4.22 gehören zur vorliegenden Erfindung nicht.

### Beispiel 4.1 N-[4-(7-Acetylamino-3-pyridin-3-yl-4,5-dihydro-pyrazolo[4,3-g]benzthiazol-1-yl)-3-fluorphenyl]-2-morpholin-4-yl-acetamid

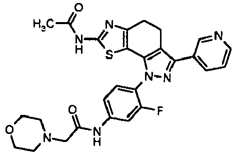

Eine Lösung von IV-1 (50 mg, 0.1 mmol) und Morpholin (0.1 mL, 1.1 mmol) in 1 mL DMF wird in der Mikrowelle (CEM) 10 min bei 100 °C gerührt. Anschließend wird die Reaktionsmischung mit wenig DMSO versetzt und an RP-Kieselgel chromatographisch gereinigt. Ausbeute: 33 mg.
HPLC: Rt = 1.32 min
[M+1]⁺ = 548

### Beispiele 4.2 - 4.22

### Analog zu Beispiel 4.1 werden Beispiele 4.2 - 4.22 synthetisiert

| # | Edukt | Struktur | Masse [M+1]⁺ | HPLC Rt [min] |
|---|---|---|---|---|
| 4.2 | IV-1 | 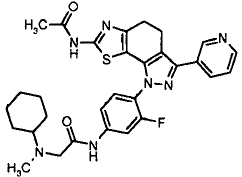 | 574 | 1.41 |
| 4.3 | IV-1 | 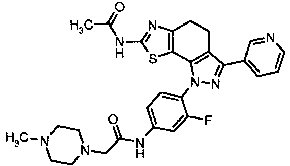 | 561 | 1.33 |
| 4.4 | IV-1 | 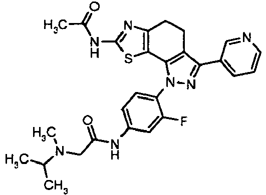 | 534 | 1.35 |
| 4.5 | IV-1 | 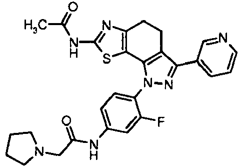 | 532 | 1.36 |
| 4.6 | IV-1 | 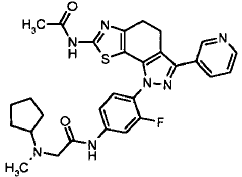 | 560 | 1.43 |
| 4.8* | IV-3 | 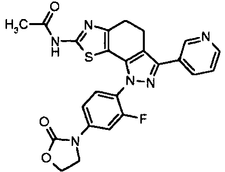 | 491 | 1.44 |
| 4.9** | IV-4 | 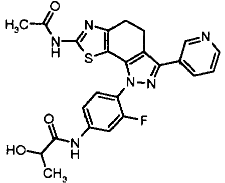 | 493 | 1.47 |
| 4.10 | IV-4 | 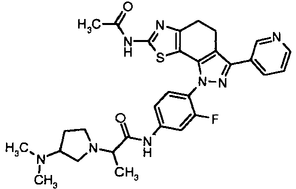 | 589 | 1.2 |
| 4.11 | IV-4 | 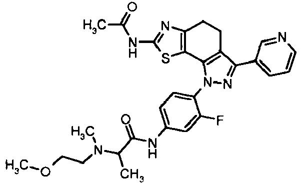 | 564 | 1.32 |
| 4.13 | IV-4 | 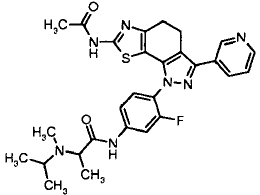 | 548 | 1.31 |
| 4.14 | IV-4 | 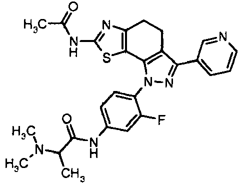 | 520 | 1.31 |
| 4.15 | IV-4 | 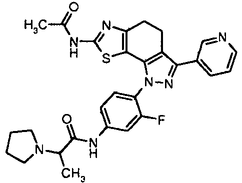 | 546 | 1.32 |
| 4.16 | IV-4 | 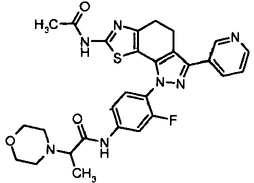 | 562 | 1.28 |
| 4.17 | IV-2 | 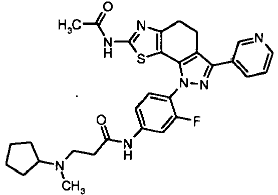 | 574 | 1.39 |
| 4.18 | IV-2 | 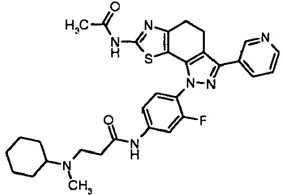 | 588 | 1.43 |
| 4.19 | IV-2 | 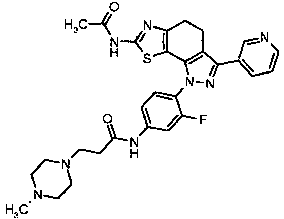 | 575 | 1.29 |
| 4.20 | IV-2 | 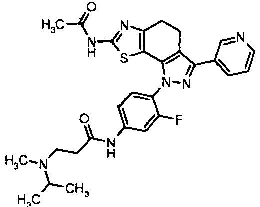 | 548 | 1.4 |
| 4.21 | IV-2 | 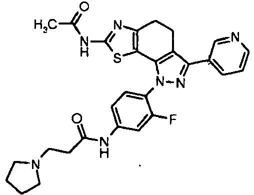 | 546 | 1.36 |
| 4.22 | IV-2 | 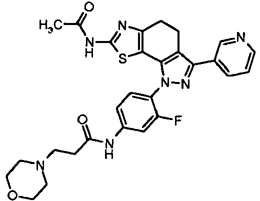 | 562 | 1.34 |

| | | | | |
|---|---|---|---|---|
| * Beispiel 4.8 wird als Nebenprodukt aus der Umsetzung von IV-3 mit Dimethylamin erhalten. ** Beispiel 4.9 wird als Nebenprodukt aus der Umsetzung von IV-4 mit *N*,*O-*Dimethylhydroxylamin Hydrochlorid erhalten. | | | | |

Analog zu den vorstehend beschriebenen Beispielen werden folgende Substanzen synthetisiert. Die Verbindungen, die in Seiten S3 und S4 dargestellt werden, gehören zur vorliegenden Erfindung nicht.

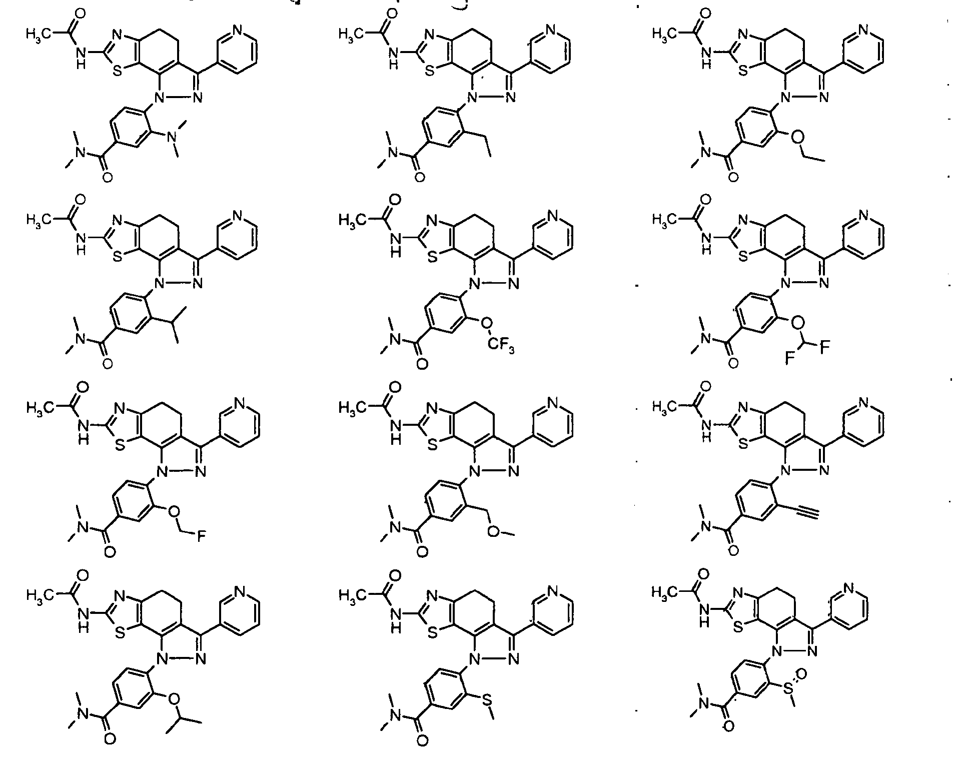

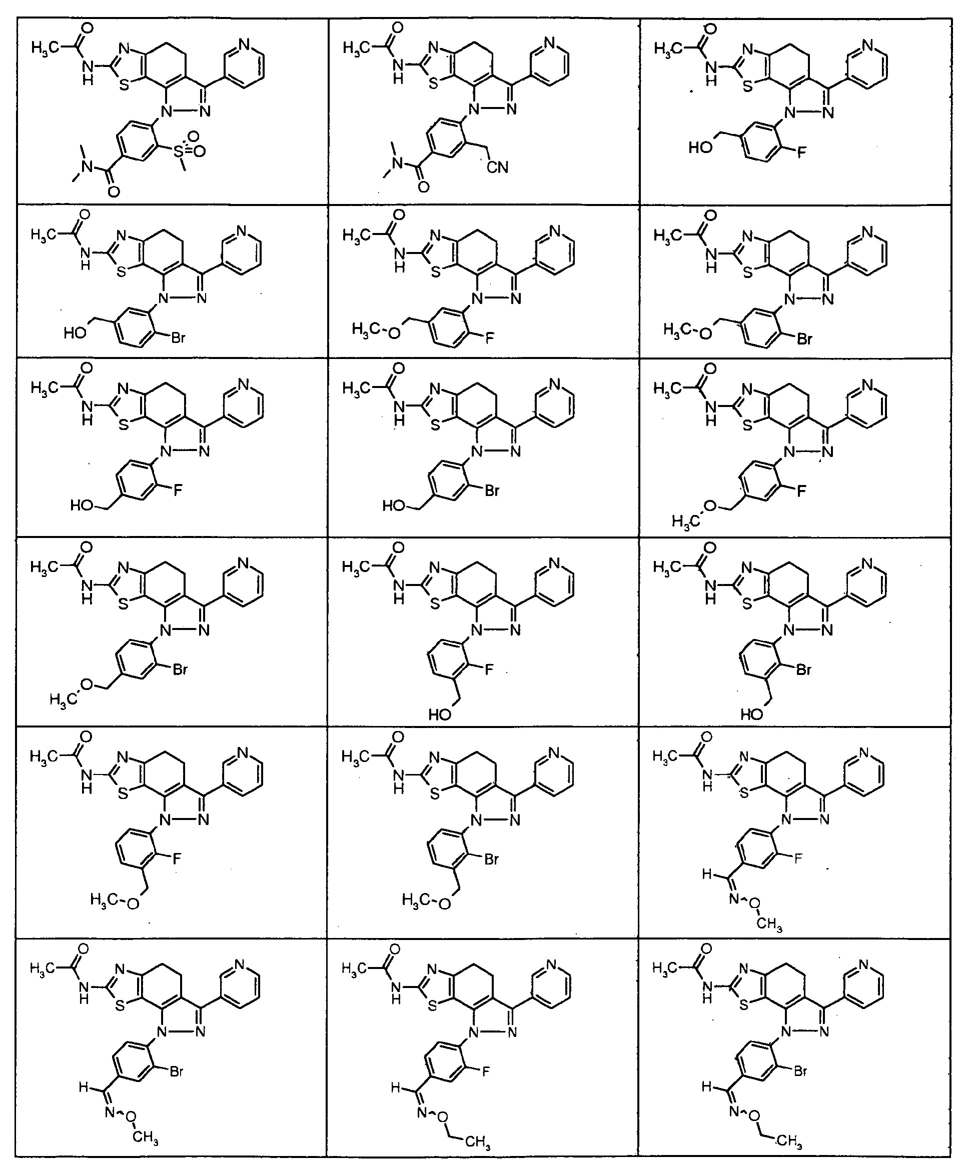

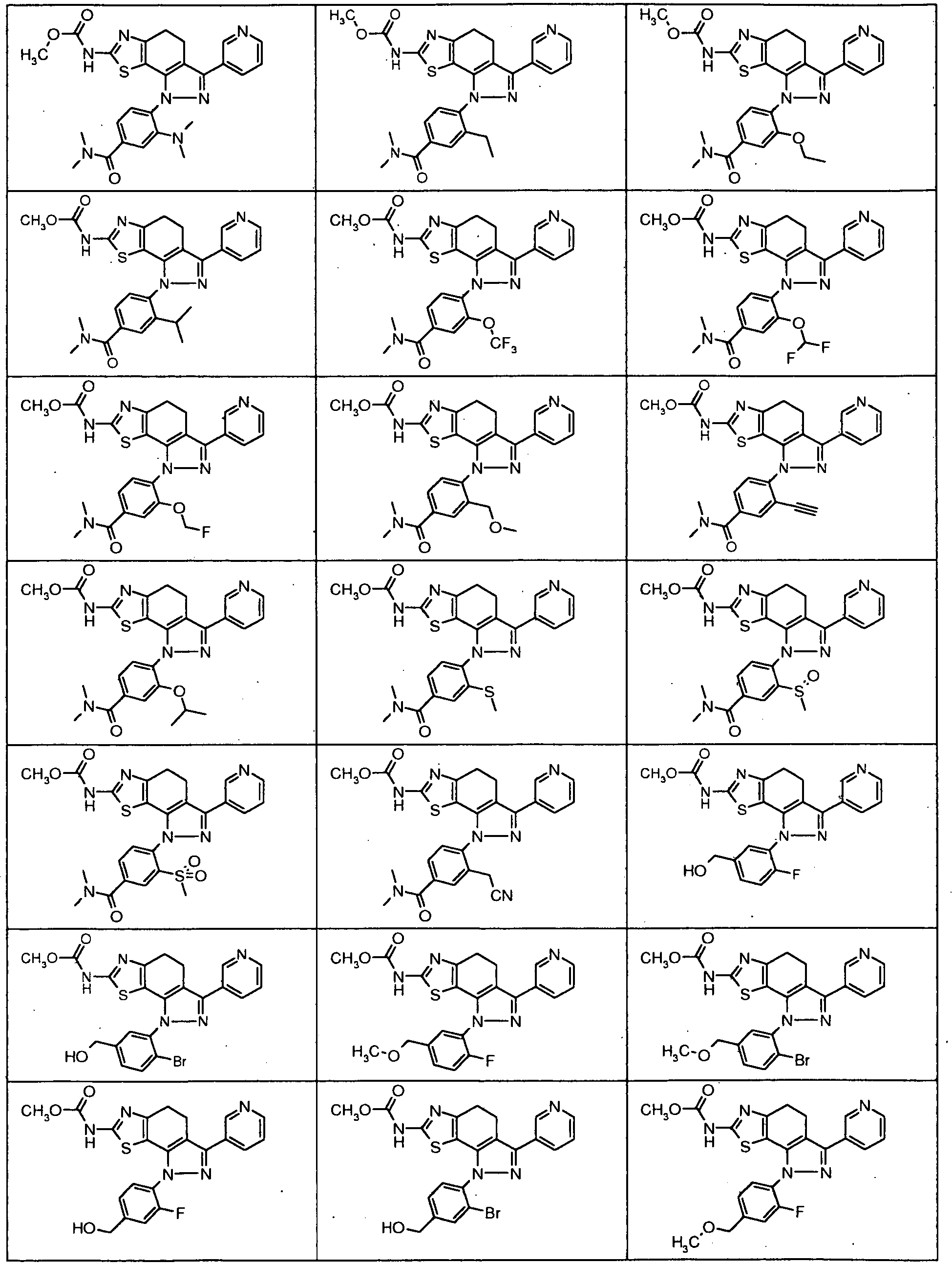

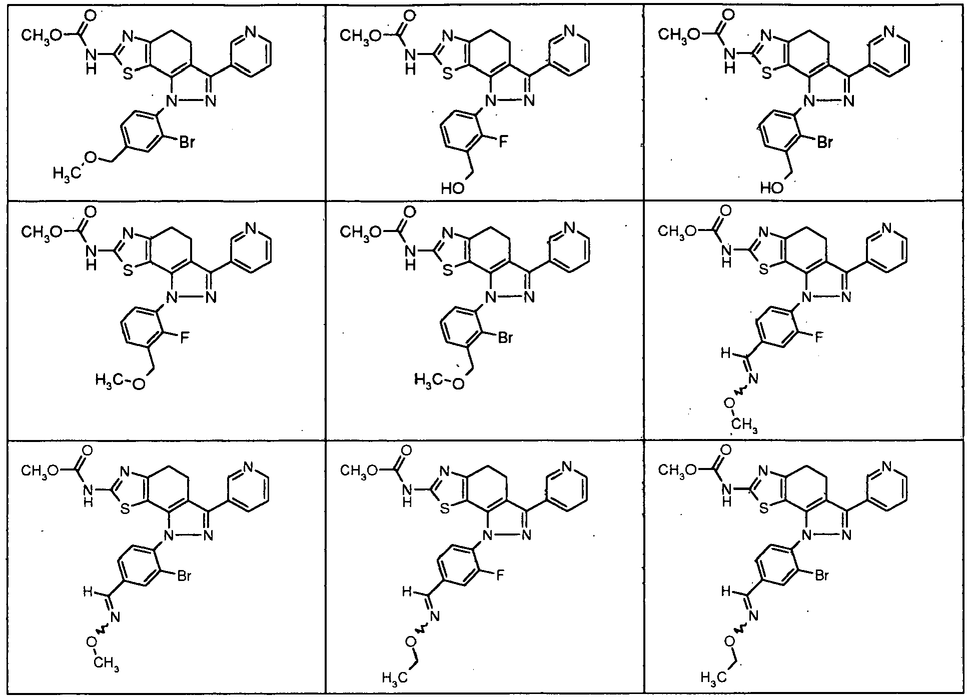

Das nachfolgende Beispiel beschreibt die biologische Wirkung der erfindungsgemäßen Verbindungen ohne die Erfindung auf dieses Beispiel einzuschränken.

### HCT116 Zytotoxizitätstest

Der Test basiert auf der Reduktion von AlamarBlue (Biosource Int., USA) in lebenden (metabolisch aktiven) Zellen zu einem fluorometrisch nachweisbaren Produkt. In Gegenwart von zelltoxischen Substanzen kann das Substrat nicht mehr reduziert werden, so dass kein Anstieg der Fluoreszenz messbar ist.

HCT116 (humane Kolonkarzinomzelllinie) Zellen werden in Mikrotiterplatten ausgesät und über Nacht in Kulturmedium bei 37 °C und 5% CO₂ inkubiert. Die Testsubstanzen werden in Medium schrittweise verdünnt und zu den Zellen gegeben, so dass das Gesamtvolumen 200 µL/well beträgt. Als Kontrollen dienen Zellen, die mit Medium, jedoch nicht mit Substanz, versetzt werden. Nach einer Inkubationszeit von 4 - 6 Tagen gibt man 20 µL/well AlamarBlue zu und inkubiert die Zellen für weitere 6 - 8 h bei 37 °C. Zur Fluoreszenzmessung wird bei einer Wellenlänge von 545 nm angeregt und bei 590 nm die Emission gemessen.

Die Berechnung von EC₅₀ Werten erfolgt mit Hilfe des Programms GraphPad Prism. Alle Verbindungen der angeführten Beispiele 1.1 bis 4.22 weisen einen EC₅₀ (HCT-116) von kleiner als 5 µM auf.

Die Substanzen der vorliegenden Erfindung sind PI3-Kinase Inhibitoren. Aufgrund ihrer biologischen Eigenschaften eignen sich die neuen Verbindungen der allgemeinen Formel (1), deren Isomere und deren physiologisch verträgliche Salze zur Behandlung von Erkrankungen, die durch exzessive oder anomale Zellproliferation charakterisiert sind.

Zu solchen Erkrankungen gehören beispielsweise: Virale Infektionen (z.B. HIV und Kaposi Sarkoma); Entzündung und Autoimmun-Erkrankungen (z.B. Colitis, Arthritis, Alzheimer Erkrankung, Glomerulonephritis und Wund-Heilung); bakterielle, fungale und/oder parasitäre Infektionen; Leukämien, Lymphome und solide Tumore; HautErkrankungen (z.B. Psoriasis); Knochen-Erkrankungen; kardiovaskuläre Erkrankungen (z.B. Restenose und Hypertrophie). Ferner sind sie nützlich als Schutz von proliferierenden Zellen (z.B. Haar-, Intestinal-, Blut- und Progenitor-Zellen) gegen DNA-Schädigung durch Strahlung, UV-Behandlung und/oder zytostatischer Behandlung (Davis et al., 2001).

Beispielsweise können folgende Krebserkrankungen mit erfindungsgemäßen Verbindungen behandelt werden, ohne jedoch darauf beschränkt zu sein: Hirntumore wie beispielsweise Akustikusneurinom, Astrozytome wie pilozytisches Astrozytome, fibrilläres Astrozytom, protoplasmatisches Astrozytom, gemistozytäres Astrozytom, anaplastisches Astrozytom und Glioblastome, Hirnlymphome, Hirnmetastasen, Hypophysentumor wie Prolaktinom, HGH (human growth hormon) produzierender Tumor und ACTH produzierender Tumor (adrenocorticotropes Hormon), Kraniopharyngiome, Medulloblastome, Meningeome und Oligodendrogliome; Nerventumore (Neoplasmen) wie zum Beispiel Tumore des vegetativen Nervensystems wie Neuroblastoma sympathicum, Ganglioneurom, Paragangliom (Phäochromozytom, Chromaffinom) und Glomuscaroticum-Tumor, Tumore am peripheren Nervensystem wie Amputationsneurom, Neurofibrom, Neurinom (Neurilemmom, Schwannom) und malignes Schwannom, sowie Tumore am zentralen Nervensystem als Hirn- und Rückenmarkstumoren; Darmkrebs wie zum Beispiel Rektumkarzinom, Kolonkarzinom, Analkarzinom, Dünndarmtumore und Zwölffingerdarmtumore; Lidtumore wie Basaliom oder Basalzellkarzinom; Bauchspeicheldrüsenkrebs oder Pankreaskarzinom; Blasenkrebs oder Blasenkarzinom; Lungenkrebs (Bronchialkarzinom) wie beispielsweise kleinzellige Bronchialkarzinome (Haferzellkarzinome) und nicht-kleinzellige Bronchialkarzinome wie Plattenepithelkarzinome, Adenokarzinome und großzellige Bronchialkarzinome; Brustkrebs wie zum Beispiel Mammakarzinom wie infiltrierend duktales Karzinom, Kolloidkarzinom, lobulär invasives Karzinom, tubuläres Karzinom, adenozystisches Karzinom und papilläres Karzinom; Non-Hodgkin-Lymphomen (NHL) wie beispielsweise Burkitt-Lymphom, niedrigmaligne Non-Hodgkin-Lymphomen (NHL) und Mucosis fungoides; Gebärmutterkrebs oder Endometriumkarzinom bzw. Corpuskarzinom; CUP-Syndrom (Cancer of Unknown Primary); Eierstockskrebs oder Ovarial-Karzinom wie mucinöse, endometriale oder seröse Krebs; Gallenblasenkrebs; Gallengangskrebs wie beispielsweise Klatskin-Tumor; Hodenkrebs wie zum Beispiel Seminome und Nicht-Seminome; Lymphom (Lymphosarkom) wie zum Beispiel malignes Lymphom, Morbus Hodgkin, Non-Hodgkin-Lymphomen (NHL) wie chronisch lymphatische Leukämie, Haarzell-Leukämie, Immunozytom, Plasmozytom (multiples Myelom), Immunoblastom, Burkitt-Lymphom, T-Zonen-Mycosis fungoides, großzellig-anaplastisches Lymphoblastom und Lymphoblastom; Kehlkopfkrebs wie zum Beispiel Stimmbandtumoren, supraglottisch, glottisch und subglottisch Kehlkopftumore; Knochenkrebs wie zum Beispiel Osteochondrom, Chondrom, Chondroblastom, Chondromyxoidfibrom, Osteom, Osteoid-Osteom, Osteoblastom, eosinophiles Granulom, Riesenzell-Tumor, Chondrosarkom, Osteosarkom, Ewing-Sarkom, Retikulo-Sarkom, Plasmozytom, Riesenzell-Tumor, fibröse Dysplasie, juvenile Knochenzyste und aneurysmatische Knochenzyste; Kopf-Hals-Tumoren wie zum Beispiel Tumore der Lippen, Zunge, Mundboden, Mundhöhle Zahnfleisch, Gaumen, Speicheldrüsen, Rachen, Nasenhöhlen, Nasennebenhöhlen, Kehlkopf und Mittelohr; Leberkrebs wie zum Beispiel das Leberzellkarzinom oder hepatozelluläres Karzinom (HCC); Leukämien wie zum Beispiel akute Leukämien wie akute lymphatische/lymphoblastische Leukämie (ALL), akute myeloische Leukämie (AML); chronische Leukämien wie chronisch lymphatische Leukämie (CLL), chronisch myeloische Leukämie (CML); Magenkrebs oder Magenkarzinom wie zum Beispiel papilläres, tubuläres und muzinöses Adenokarzinom, Siegelringzellkarzinom adenosquamöses Karzinom, kleinzelliges Karzinom und undifferenziertes Karzinom; Melanome wie zum Beispiel oberflächlich spreitendes, knotiges, lenitgo-maligna und akral-lentiginöse Melanom; Nierenkrebs wie zum Beispiel Nierenzellkarzinom oder Hypernephrom oder Grawitz-Tumor; Speiseröhrenkrebs oder Ösophaguskarzinom; Peniskrebs; Prostatakrebs; Rachenkrebs oder Pharynxkarzinome wie zum Beispiel Nasopharynxkarzinome, Oropharynxkarzinome und Hypopharynxkarzinome; Retinoblastom; Scheidenkrebs oder Vaginalkarzinom; Plattenepithelkarzinome, Adenokarzinome, in situ Karzinome, maligne Melanome und Sarkome; Schilddrüsen-Karzinome wie zum Beispiel papilläre, follikuläre und medulläre Schilddrüsen-Karzinom, sowie anaplastische Karzinome; Spinaliom, Stachelzellkarzinom und Plattenepithelkarzinom der Haut; Thymome, Urethrakrebs und Vulvakrebs.

Die neuen Verbindungen können zur Prävention, Kurz- oder Langzeitbehandlung der vorstehend erwähnten Krankheiten auch gegebenenfalls in Kombination mit anderen "State-of-art" Verbindungen, wie anderen anti-Tumor Substanzen, zytotoxischen Substanzen, Zellproliferationshemmern, anti-angiogenen Substanzen, Steroiden oder Antikörpern, verwendet werden.

Die Verbindungen der allgemeinen Formel (1) können allein oder in Kombination mit anderen erfindungsgemäßen Wirkstoffen, gegebenenfalls auch in Kombination mit weiteren pharmakologisch aktiven Wirkstoffen, zur Anwendung gelangen. Chemotherapeutika, welche in Kombination mit den erfindungsgemäßen Verbindungen verabreicht werden können, umfassen, ohne darauf eingeschränkt zu sein, Hormone, Hormonanaloge und Antihormone (z.B. Tamoxifen, Toremifen, Raloxifen, Fulvestrant, Megestrol Acetat, Flutamid, Nilutamid, Bicalutamid, Aminoglutethimid, Cyproteron Acetat, Finasterid, Buserelin Acetat, Fludrocortinson, Fluoxymesteron, Medroxyprogesteron, Octreotid), Aromatase Inhibitoren (z.B. Anastrozol, Letrozol, Liarozol, Vorozol, Exemestan, Atamestan,), LHRH Agonisten und Antagonists (z.B. Goserelin Acetat, Luprolid), Inhibitoren von Wachstumsfaktoren (growth factors wie zum Beispiel "platelet derived growth factor" und "hepatocyte growth factor", Inhibitoren sind z. B. "growth factor"-Antikörper, "growth factor receptor"-Antikörper und Tyrosimekinase Inhibitoren, wie zum Beispiel Gefitinib, Imatinib, Lapatinib, Cetuximab (Erbitux®) und Trastuzumab); Antimetabolite (z.B. Antifolate wie Methotrexat, Raltitrexed, Pyrimidinanaloge wie 5-Fluorouracil, Capecitabin und Gemcitabin, Purin- und Adenosinanaloge wie Mercaptopurin, Thioguanin, Cladribin und Pentostatin, Cytarabin, Fludarabin); Antitumor-Antibiotica (z.B. Anthracycline wie Doxorubicin, Daunorubicin, Epirubicin und Idarubicin, Mitomycin-C, Bleomycin, Dactinomycin, Plicamycin, Streptozocin); Platinderivative (z.B. Cisplatin, Oxaliplatin, Carboplatin); Alkylierungsagentien (z.B. Estramustin, Meclorethamin, Melphalan, Chlorambucil, Busulphan, Dacarbazin, Cyclophosphamid, Ifosfamid, Temozolomid, Nitrosoharnstoffe wie zum Beispiel Carmustin und Lomustin, Thiotepa); antimitotische Agentien (z.B. Vinca-Alkaloide wie zum Beispiel Vinblastin, Vindesin, Vinorelbin und Vincristin; und Taxane wie Paclitaxel, Docetaxel); Topoisomerase Inhibitoren (z.B. Epipodophyllotoxine wie zum Beispiel Etoposid und Etopophos, Teniposid, Amsacrin, Topotecan, Irinotecan, Mitoxantron) und verschiedene Chemotherapeutica wie Amifostin, Anagrelid, Clodronat, Filgrastin, Interferon alpha, Leucovorin, Rituximab, Procarbazin, Levamisol, Mesna, Mitotan, Pamidronat und Porfimer.

Geeignete Anwendungsformen sind beispielsweise Tabletten, Kapseln, Zäpfchen, Lösungen, - insbesondere Lösungen zur Injektion (s.c., i.v., i.m.) und Infusion - Säfte, Emulsionen oder dispersible Pulver. Hierbei soll der Anteil der pharmazeutisch wirksamen Verbindung(en) jeweils im Bereich von 0,1 - 90 Gew.-%, bevorzugt 0,5 - 50 Gew.-% der Gesamtzusammensetzung liegen, das heißt in Mengen die ausreichend sind, um den unten angegebenen Dosierungsbereich zu erreichen. Die genannten Dosen können, falls erforderlich, mehrmals täglich gegeben werden.

Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektions- und Infusionslösungen werden in üblicher Weise, z.B. unter Zusatz von Isotonantien, Konservierungsmitteln, wie p-Hydroxybenzoate, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilfslösemittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Die Applikation erfolgt in üblicher Weise, vorzugsweise oral oder transdermal, insbesondere bevorzugt oral. Im Falle der oralen Anwendung können die Tabletten selbstverständlich außer den genannten Trägerstoffen auch Zusätze, wie z.B. Natriumcitrat, Calciumcarbonat und Dikalziumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wässriger Suspensionen können die Wirkstoffe außer den oben genannten Hilfsstoffen mit verschiedenen Geschmacksaufbesserem oder Farbstoffen versetzt werden.

Für den Fall der parenteralen Anwendung können Lösungen der Wirkstoffe unter Verwendung geeigneter flüssiger Trägermaterialien eingesetzt werden.
Die Dosierung für die intravenöse Anwendung liegt bei 1 - 1000 mg pro Stunde, vorzugsweise zwischen 5 - 500 mg pro Stunde.

Trotzdem kann es gegebenenfalls notwendig sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Formulierungsbeispiele illustrieren die vorliegende Erfindung ohne sie jedoch in ihrem Umfang zu beschränken:

### Pharmazeutische Formulierungsbeispiele

| A) Tabletten | pro Tablette |
|---|---|
| Wirkstoff nach Formel (1) | 100 mg |
| Milchzucker | 140 mg |
| Maisstärke | 240 mg |
| Polyvinylpyrrolidon | 15 mg |
| Magnesiumstearat | 5 mg |
| | 500 mg |

Der fein gemahlene Wirkstoff, Milchzucker und ein Teil der Maisstärke werden miteinander vermischt. Die Mischung wird gesiebt, danach mit einer Lösung von Polyvinylpyrrolidon in Wasser befeuchtet, geknetet, feucht granuliert und getrocknet. Das Granulat, der Rest der Maisstärke und das Magnesiumstearat werden gesiebt und miteinander vermischt. Das Gemisch wird zu Tabletten geeigneter Form und Größe verpresst.

| B) Tabletten | pro Tablette |
|---|---|
| Wirkstoff nach Formel (1) | 80 mg |
| Milchzucker | 55 mg |
| Maisstärke | 190 mg |
| Mikrokristalline Cellulose | 35 mg |
| Polyvinylpyrrolidon | 15 mg |
| Natrium-carboxymethylstärke | 23 mg |
| Magnesiumstearat | 2 mg |
| | 400 mg |

Der fein gemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpresst das Gemisch zu Tabletten geeigneter Größe.

| C) Ampullenlösung | |
|---|---|
| Wirkstoff nach Formel (1) | 50 mg |
| Natriumchlorid | 50 mg |
| Aqua pro inj. | 5 ml |

Der Wirkstoff wird bei Eigen-pH oder gegebenenfalls bei pH 5,5 - 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

## Patentansprüche

1. Verbindungen der allgemeinen Formel (1), 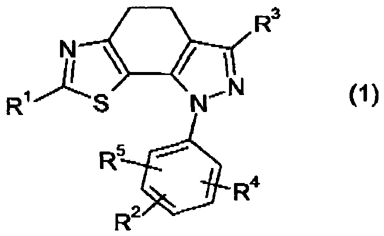 worin
**R¹** ausgewählt aus der Gruppe bestehend aus -NHC(O)OR^{c}, und
**R²** C₁₋₆Alkyl oder 3-8 gliedriges Heterocycloalkyl, gegebenenfalls substituiert mit einem oder mehreren R⁶, oder ein Rest ausgewählt aus der Gruppe bestehend aus Halogen, -NO₂, -NR^{c}R^{c}. -OR^{c}, - S(O)R^{c}, -S(O)₂R^{c}, -C(O)R^{c}, -C(O)OR^{c}, -C(O)NR^{c}R^{c}, -C(O)N(R^{g})OR^{c} -N(R^{g})C(O)R^{c}, -N(R^{g})C(O)OR^{c}, -NR^{g}C(O)NR^{c}R^{c} -N(R^{g})C(O)SR^{c} und -N(R^{g})S(O)₂R^{c}, und
**R³** ein Rest ausgewählt aus der Gruppe bestehend aus C₆₋₁₀Aryl und 5-6 gliedriges Heteroaryl, gegebenenfalls substituiert mit einem oder mehreren, gleich oder verschiedenen R^{c} und/oder R^{b}, und
**R⁴** ein Rest ausgewählt aus der Gruppe bestehend aus Brom, Fluor, -CF₃, -OCF₃, -CN, -NR^{c}R^{c}, -SR^{c}, -S(O)R^{c}, -S(O)₂R^{c} und -OR^{c}, oder C₁₋₃Alkyl gegebenenfalls substituiert mit Fluor, -CN, -NR^{f}R^{f} und/oder -OR^{f}; und
**R⁵** Wasserstoff oder ein Rest ausgewählt aus der Gruppe bestehend aus Halogen, -CF₃, -OCF₃, -CN, -NR^{c}R^{c}, -SR^{c}, -S(O)R^{c}, -S(O)₂R^{c} und -OR^{c}, oder C₁₋₃Alkyl gegebenenfalls substituiert mit Fluor, -CN, -NR^{f}R^{f} und/oder -OR^{f}, und
**R⁶** ein Rest ausgewählt aus der Gruppe bestehend aus R^{a}, R^{b} und R^{a} substituiert mit einem oder mehreren, gleich oder verschiedenen R^{c} und/oder R^{b}, und
jedes **R^{a}** unabhängig voneinander ausgewählt aus der Gruppe bestehend aus C₁₋₆Alkyl, C₃₋₈Cycloalkyl, C₄₋₁₁Cycloalkylalkyl, C₆₋₁₀aryl, C₇₋₁₆Arylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl, und
jedes **R^{b}** ein geeigneter Rest und jeweils unabhängig voneinander ausgewählt aus der Gruppe bestehend aus =O, -OR^{c}, C₁₋₃Haloakyloxy, -OCF₃, =S, -SR^{c}, -NR^{c}, =NOR^{c}, -NR^{c}R^{c}, Halogen, -CF₃, -CN, -NC, -NO₂, -N₃, -S(O)R^{c}, -S(O)₂R^{c}, -S(O)₂OR^{c}, - S(O)NR^{c}R^{c}, -S(O)₂NR^{c}R^{c}, -OS(O)R^{c}, -OS(O)₂R^{c}, -OS(O)₂OR^{c}, -OS(O)₂NR^{c}R^{c}, - C(O)R^{c}, -C(O)OR^{c}, -C(O)NR^{c}R^{c}, -C(O)N(R^{g})NR^{c}R^{c}, -C(O)N(R^{g})OR^{c}, -CN(R^{g})NR^{c}R^{c}, -OC(O)R^{c}, -OC(O)OR^{c}, -OC(O)NR^{c}R^{c}, -N(R^{g})C(O)R^{c}, -N(R^{g})C(O)R^{c}, -N(R^{g})C(S)R^{c}, -N(R^{g})S(O)₂R^{c}, -N(R^{g})S(O)₂NR^{c}R^{c}, -N(R^{g})C(O)OR^{c}, -N(R^{g})C(O)NR^{c}R^{c}, und -N(R^{g})CN(R^{g})NR^{c}R^{c}, und
jedes **R^{c}** unabhängig voneinander Wasserstoff oder ein gegebenenfalls mit einem oder mehreren, gleich oder verschiedenen R^{d} und/oder R^{e} substituierter Rest ausgewählt aus der Gruppe bestehend aus C₁₋₆Alkyl, C₃₋₈Cycloalkyl, C₄₋₁₁Cycloalkylakyl, C₆₋₁₀Aryl, C₇₋₁₆Arylalkyl, 2-6 gliedrigcs Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl, und
jedes **R^{d}** unabhängig voneinander ein gegebenenfalls mit einem oder mehreren, gleich oder verschiedenen R^{c} und/oder R^{f} substituierter Rest ausgewählt aus der Gruppe bestehend aus C₁₋₆Alkyl, C₁₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl, und
jedes **R^{e}** ein geeigneter Rest und jeweils unabhängig voneinander ausgewählt aus der Gruppe bestehend aus -O, -OR^{f}, C₁₋₃Haloalkyloxy, -OCF₃, =S, -SR^{f}, =NR^{f}, =NOR^{f}, -NR^{f}R^{f}, Halogen, -CF₃, -CN, -NC, -NO₂, -N₃, -S(O)R^{f}, -S(O)₂R^{f}, -S(O)₂OR^{f}, - S(O)NR^{f}R^{f}, -S(O)₂NR^{f}R^{f}, -OS(O)R^{f}, -OS(O)₂R^{f}; -OS(O)₂OR^{f}; -OS(O)₂NR^{f}R^{f}, - C(O)R^{f}, -C(O)OR^{f}, -C(O)NR^{f}R^{f}; -C(O)N(R^{g})OR^{f} -CN(R^{g})NR^{f}R^{f}, -OC(O)R^{f}, -OC(O)OR^{f}, -OC(O)NR^{f}R^{f}, -OCN(R^{g})NR^{f}R^{f}, -N(R^{g})C(O)R^{f}, -N(R^{g})C(S)R^{f}; - N(R^{g})S(O)₂R^{f}, -N(R^{g})C(O)OR^{f}, -N(R^{g})C(O)NR^{f}R^{f}, und -N(R^{g})CN(R^{g})NR^{f}R^{f}, und jedes **R^{f}** unabhängig voneinander Wasserstoff oder ein gegebenenfalls mit einem oder mehreren, gleich oder verschiedenen R^{g} substituierter Rest ausgewählt aus der Gruppe bestehend aus C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl, und
jedes **R^{g}** unabhängig voneinander Wasserstoff, C₁₋₆Alkyl, C₃₋₈Cycloalkyl, C₄₋₁₁Cycloalkylalkyl, C₆₋₁₀Aryl, C₇₋₁₆Arylalkyl, 2-6 gliedriges Heteroalkyl, 3-8 gliedriges Heterocycloalkyl, 4-14 gliedriges Heterocycloalkylalkyl, 5-10 gliedriges Heteroaryl und 6-16 gliedriges Heteroarylalkyl bedeuten, gegebenenfalls in Form ihrer Tautomeren, ihrer Racemate, ihrer Enantiomere, ihrer Diastereomere und ihrer Gemische, sowie gegebenenfalls ihrer pharmakologisch verträglichen Salze.

2. Verbindungen gemäß Anspruch 1 der allgemeinen Formel (1A), 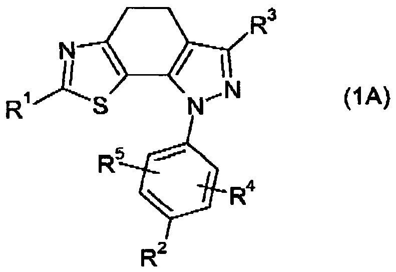 worin die Substituenten wie in Anspruch 1 definiert sind.

3. Verbindungen gemäß Anspruch 1 der allgemeinen Formel (1B), 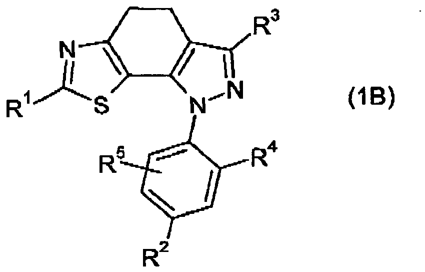 worin die Substituenten wie in Anspruch 1 definiert sind.

4. Verbindungen gemäß einem der Ansprüche 1 bis 3, worin R³ 5-6 gliedriges Heteroaryl, gegebenenfalls substituiert mit einem oder mehreren, gleich oder verschiedenen R^{c} und/oder R^{b} bedeutet.

5. Verbindungen gemäß einem der Ansprüche 1 bis 4, worin **R³** unsubstituiertes Pyridyl bedeutet.

6. Verbindungen, oder deren pharmakologisch verträglichen Salze, gemäß einem der Ansprüche 1 bis 5 als Arzneimittel.

7. Verbindungen, oder deren pharmakologisch verträglichen Salze, gemäß einem der Ansprüche 1 bis 5 zur Herstellung eines Arzneimittels mit antiproliferativer Wirkung.

8. Pharmazeutische Zubereitungen, enthaltend als Wirkstoff eine oder mehrere Verbindungen der allgemeinen Formel (1) gemäß einem der Ansprüche 1 bis 7 oder deren pharmakologisch verträglichen Salze, gegebenenfalls in Kombination mit üblichen Hilfs- und/oder Trägerstoffen.

9. Verwendung von Verbindungen der allgemeinen Formel (1) gemäß einem der Ansprüche 1 bis 7 zur Herstellung eines Arzneimittels zur Behandlung und/oder Prävention von Krebs.

10. Pharmazeutische Präparation umfassend eine Verbindung der allgemeinen Formel (1) gemäß einem der Ansprüche 1 bis 7 und mindestens eine weitere zytostatische oder zytotoxische, von Formel (1) verschiedene Wirksubstanz, gegebenenfalls in Form ihrer Tautomeren, ihrer Racemate, ihrer Enantiomere, ihrer Diastereomere und ihrer Gemische, sowie gegebenenfalls ihrer pharmakologisch verträglichen Salze.

## Claims

1. Compounds of general formula (1), 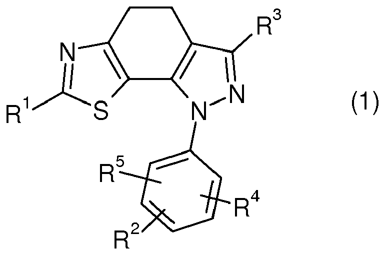 wherein
**R¹** is selected from among -NHC(O)OR^{c}, and
**R²** denotes C₁₋₆alkyl or 3-8 membered heterocycloalkyl, optionally substituted by one or more R⁶, or a group selected from among halogen, -NO₂, -NR^{c}R^{c}, -OR^{c}, -S(O)R^{c}, -S(O)₂R^{c}, -C(O)R^{c}, -C(O)OR^{c}, -C(O)NR^{c}R^{c}, -C(O)N(R^{g})OR^{c} -N(R^{g})C(O)R^{c}, -N(R^{g})C(O)OR^{c}, -NR^{g}C(O)NR^{c}R^{c} -N(R^{g})C(O)SR^{c} and -N(R^{g})S(O)₂R^{c}, and
**R³** denotes a group selected from among C₆₋₁₀aryl and 5-6 membered heteroaryl, optionally substituted by one or more, identical or different R^{c} and/or R^{b}, and
**R⁴** denotes a group selected from among bromine, fluorine, -CF₃, -OCF₃, -CN, -NR^{c}R^{c}, -SR^{c}, -S(O)R^{c}, -S(O)₂R^{c} and -OR^{c}, or C₁₋₃alkyl optionally substituted by fluorine, -CN, -NR^{f}R^{f} and/or -OR^{f}, and
**R⁵** denotes hydrogen or a group selected from among halogen, -CF₃, -OCF₃, -CN, -NR^{c}R^{c}, -SR^{c}, -S(O)R^{c}, -S(O)₂R^{c} and -OR^{c}, or C₁₋₃alkyl optionally substituted by fluorine, -CN, -NR^{f}R^{f} and/or -OR^{f}, and
**R⁶** denotes a group selected from among R^{a}, R^{b} and R^{a} substituted by one or more, identical or different R^{c} and/or R^{b}, and
each **R^{a}** independently of one another is selected from among C₁₋₆alkyl, C₃₋₈cycloalkyl, C₄₋₁₁cycloalkylalkyl, C₆₋₁₀aryl, C₇₋₁₆arylalkyl, 2-6 membered heteroalkyl, 3-8 membered heterocycloalkyl, 4-14 membered heterocycloalkylalkyl, 5-10 membered heteroaryl and 6-16 membered heteroarylalkyl, and
each **R^{b}** denotes a suitable group each independently selected from among =O, -OR^{c}, C₁₋₃haloalkyloxy, -OCF₃, =S, -SR^{c}, =NR^{c}, =NOR^{c}, -NR^{c}R^{c}, halogen, -CF₃, -CN, -NC, -NO₂, -N₃, -S(O)R^{c}, -S(O)₂R^{c}, -S(O)₂OR^{c}, -S(O)NR^{c}R^{c}, -S(O)₂NR^{c}R^{c}, -OS(O)R^{c}, -OS(O)₂R^{c}, -OS(O)₂OR^{c}, -OS(O)₂NR^{c}R^{c}, -C(O)R^{c}, -C(O)OR^{c}, -C(O)NR^{c}R^{c}, -C(O)N(R^{g})NR^{c}R^{c}, -C(O)N(R^{g})OR^{c}, -CN(R^{g})NR^{c}R^{c}, -OC(O)R^{c}, -OC(O)OR^{c}, -OC(O)NR^{c}R^{c}, -N(R^{g})C(O)R^{c}, -N(R^{g})C(O)R^{c}, -N(R^{g})C(S)R^{c}, -N(R^{g})S(O)₂R^{c}, -N(R^{g})S(O)₂NR^{c}R^{c}, -N(R^{g})C(O)OR^{c}, -N(R^{g})C(O)NR^{c}R^{c}, and -N(R^{g})CN(R^{g})NR^{c}R^{c}, and
each **R^{c}** independently of one another denotes hydrogen or a group optionally substituted by one or more, identical or different R^{d} and/or R^{e} selected from among C₁₋₆alkyl, C₃₋₈cycloalkyl, C₄₋₁₁cycloalkylalkyl, C₆₋₁₀aryl, C₇₋₁₆arylalkyl, 2-6 membered heteroalkyl, 3-8 membered heterocycloalkyl, 4-14 membered heterocycloalkylalkyl, 5-10 membered heteroaryl and 6-16 membered heteroarylalkyl, and
each **R^{d}** independently of one another denotes a group optionally substituted by one or more, identical or different R^{e} and/or R^{f} selected from among C₁₋₆alkyl, C₃₋₈cycloalkyl, C₄₋₁₁cycloalkylalkyl, C₆₋₁₀aryl, C₇₋₁₆arylalkyl, 2-6 membered heteroalkyl, 3-8 membered heterocycloalkyl, 4-14 membered heterocycloalkylalkyl, 5-10 membered heteroaryl and 6-16 membered heteroarylalkyl, and
each **R^{e}** denotes a suitable group each independently selected from among =O, -OR^{f}, C₁₋₃haloalkyloxy, -OCF₃, =S, -SR^{f}, =NR^{f}, =NOR^{f}, -NR^{f}R^{f}, halogen, -CF₃, -CN, -NC, -NO₂, -N₃, -S(O)R^{f}, -S(O)₂R^{f}, -S(O)₂OR^{f}, -S(O)NR^{f}R^{f}, -S(O)₂NR^{f}R^{f}, -OS(O)R^{f}, -OS(O)₂R^{f}, -OS(O)₂OR^{f}, -OS(O)₂NR^{f}R^{f}, -C(O)R^{f}, -C(O)OR^{f}, -C(O)NR^{f}R^{f}, -C(O)N(R^{g})OR^{f} -CN(R^{g})NR^{f}R^{f}, -OC(O)R^{f}, -OC(O)OR^{f}, -OC(O)NR^{f}R^{f}, -OCN(R^{g})NR^{f}R^{f}, -N(R^{g})C(O)R^{f}, -N(R^{g})C(S)R^{f}, -N(R^{g})S(O)₂R^{f}, -N(R^{g})C(O)OR^{f}, -N(R^{g})C(O)NR^{f}R^{f}, and -N(R^{g})CN(R^{g})NR^{f}R^{f}, and
each **R^{f}** independently of one another denotes hydrogen or a group optionally substituted by one or more, identical or different R^{g} selected from among C₁₋₆alkyl, C₃₋₈cycloalkyl, C₄₋₁₁cycloalkylalkyl, C₆₋₁₀aryl, C₇₋₁₆arylalkyl, 2-6 membered heteroalkyl, 3-8 membered heterocycloalkyl, 4-14 membered heterocycloalkylalkyl, 5-10 membered heteroaryl and 6-16 membered heteroarylalkyl, and
each **R^{g}** independently of one another denotes hydrogen, C₁₋₆alkyl, C₃₋₈cycloalkyl, C₄₋₁₁cyloalkylalkyl, C₆₋₁₀aryl, C₇₋₁₆arylalkyl, 2-6 membered heteroalkyl, 3-8 membered heterocycloalkyl, 4-14 membered heterocycloalkylalkyl, 5-10 membered heteroaryl and 6-16 membered heteroarylalkyl,
optionally in the form of the tautomers, the racemates, the enantiomers, the diastereomers and the mixtures thereof, as well as optionally the pharmacologically acceptable salts thereof.

2. Compounds according to claim 1 of general formula (1A), 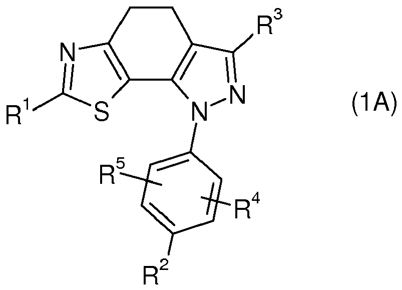 wherein the substituents are defined as in claim 1.

3. Compounds according to claim 1 of general formula (1B), 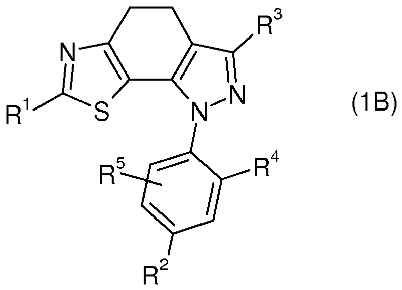 wherein the substituents are defined as in claim 1.

4. Compounds according to one of claims 1 to 3, wherein
**R³** denotes 5-6 membered heteroaryl, optionally substituted by one or more, identical or different R^{c} and/or R^{b}.

5. Compounds according to one of claims 1 to 4, wherein
**R³** denotes unsubstituted pyridyl.

6. Compounds, or the pharmacologically acceptable salts thereof, according to one of claims 1 to 5, as medicaments.

7. Compounds, or the pharmacologically acceptable salts thereof, according to one of claims 1 to 5 for preparing a medicament with an antiproliferative activity.

8. Pharmaceutical preparations, containing as active substance one or more compounds of general formula (1) according to one of claims 1 to 7 or the pharmacologically acceptable salts thereof, optionally in combination with conventional excipients and/or carriers.

9. Use of compounds of general formula (1) according to one of claims 1 to 7 for preparing a medicament for the treatment and/or prevention of cancer.

10. Pharmaceutical preparation containing a compound of general formula (1) according to one of claims 1 to 7 and at least one other cytostatic or cytotoxic active substance, different from formula (1), optionally in the form of the tautomers, the racemates, the enantiomers, the diastereomers and the mixtures thereof, as well as optionally the pharmacologically acceptable salts thereof.

## Revendications

1. Composés de formule générale (1), 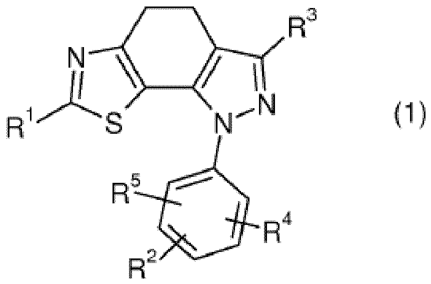 dans laquelle
R¹ est choisi dans le groupe comprenant NHC(O)OR^{c}, et R² est un groupe alkyle en C₁ à C₆ ou hétérocycloalkyle de 3 à 8 chaînons, éventuellement substitué par un ou plusieurs R⁶, ou un radical choisi dans le groupe comprenant un atome d'halogène, -NO₂, -NR^{c}R^{c}, -OR^{c}, -S(O)R^{c}, -S(O)₂R^{c}, -C(O)R^{c}, -C(O)OR^{c}, -C(O)NR^{c}R^{c}, -C(O)N(R^{g})OR^{c} -N(R^{g})C(O)R^{c}, -N(R^{g})C(O)OR^{c}, -NR^{g}C(O)NR^{c}R^{c} -N(R^{g})C(O)SR^{c} et -N(R^{g})S(O)₂R^{c}, et
R³ est un radical choisi dans le groupe comprenant un groupe aryle en C₆ à C₁₀ et hétéroaryle de 5 à 6 chaînons, éventuellement substitué par un ou plusieurs R^{c} et/ou R^{b} identiques ou différents, et
R⁴ est un radical choisi dans le groupe comprenant un atome de brome, de fluor, -CF₃, -OCF₃, -CN, -NR^{c}R^{c}, -SR^{c}, -S(O)R^{c}, -S(O)₂R^{c} et -OR^{c} ou un groupe alkyle en C₁ à C₃ éventuellement substitué par un atome de fluor, -CN, -NR^{f}R^{f} et/ou -OR^{f}, et
R⁵ est un atome d'hydrogène ou un radical choisi dans le groupe comprenant un atome d'halogène, -CF₃, -OCF₃, -CN, -NR^{c}R^{c}, -SR^{c}, -S(O)R^{c}, -S(O)₂R^{c} et -OR^{c} ou un groupe alkyle en C₁ à C₃ éventuellement substitué par un atome de fluor, -CN, -NR^{f}R^{f} et/ou -OR^{f}, et
R⁶ est un radical choisi dans le groupe comprenant R^{a},
R^{b} et R^{a} substitué par un ou plusieurs R^{c} et/ou R^{b} identiques ou différents, et
chaque R^{a}, indépendamment les uns des autres, est choisi parmi les groupes alkyle en C₁ à C₆, cycloalkyle en C₃ à C₈, cycloalkylalkyle en C₄ à C₁₁, aryle en C₆ à C₁₀, arylalkyle en C₇ à C₁₆, hétéroalkyle de 2 à 6 chaînons, hétérocycloalkyle de 3 à 8 chaînons, hétérocycloalkylalkyle de 4 à 14 chaînons, hétéroaryle de 5 à 10 chaînons et hétéroarylalkyle de 6 à 16 chaînons, et
chaque R^{b} est un radical approprié et respectivement, indépendamment les uns des autres, est choisi dans le groupe comprenant =O, -OR^{c}, un groupe halogénoalkyloxy en C₁ à C₃, -OCF₃, =S, -SR^{c}, -NR^{c}, =NOR^{c}, -NR^{c}R^{c}, halogéno, -CF₃, -CN, -NC, -NO₂, -N₃, -S(O)R^{c}, -S(O)₂R^{c}, -S(O)₂OR^{c}, -S(O)NR^{c}R^{c}, -S(O)₂NR^{c}R^{c}, -OS(O)R^{c}, -OS(O)₂R^{c}, -OS(O)₂OR^{c}, -OS(O)₂NR^{c}R^{c}, -C(O)R^{c}, -C(O)OR^{c}, -C(O)NR^{c}R^{c}, -C(O)N(R^{g})NR^{c}R^{c}, -C(O)N(R^{g})OR^{c}, -CN(R^{g})NR^{c}R^{c}, -OC(O)R^{c}, -OC(O)OR^{c}, -OC(O)NR^{c}R^{c}, -N(R^{g})C(O)R^{c}, -N(R^{g})C(O)R^{c}, -N(R^{g})C(S)R^{c}, -N(R^{g})S(O)₂R^{c}, -N(R^{g})S(O)₂NR^{c}R^{c}, -N(R^{g})C(O)OR^{c}, -N(R^{g})C(O)NR^{c}R^{c} et -N (R^{g}) CN (R^{g})NR^{c}R^{c}, et chaque R^{c}, indépendamment les uns des autres, est un atome d'hydrogène ou un radical substitué éventuellement par un ou plusieurs R^{d} et/ou R^{e} identiques ou différents choisis dans le groupe comprenant les groupes alkyle en C₁ à C₆, cycloalkyle en C₃ à C₈, cycloalkylalkyle en C₄ à C₁₁, aryle en C₆ à C₁₀, arylalkyle en C₇ à C₁₆, hétéroalkyle de 2 à 6 chaînons, hétérocycloalkyle de 3 à 8 chaînons, hétérocycloalkylalkyle de 4 à 14 chaînons, hétéroaryle de 5 à 10 chaînons et hétéroarylalkyle de 6 à 16 chaînons, et
chaque R^{d} indépendamment les uns les autres, est un radical éventuellement substitué par un ou plusieurs R^{e} et/ou R^{f} identiques ou différents choisis dans le groupe comprenant les groupes alkyle en C₁ à C₆, cycloalkyle en C₃ à C₈, cycloalkylalkyle en C₄ à C₁₁, aryle en C₆ à C₁₀, arylalkyle en C₇ à C₁₆, hétéroalkyle de 2 à 6 chaînons, hétérocycloalkyle de 3 à 8 chaînons, hétérocycloalkylalkyle de 4 à 14 chaînons, hétéroaryle de 5 à 10 chaînons et hétéroarylalkyle de 6 à 16 chaînons, et
chaque R^{e} est un radical approprié et choisi respectivement, indépendamment les uns des autres, dans le groupe comprenant -O, -OR^{f}, un groupe halogénoalkyloxy en C₁ à C₃, -OCF₃, =S, -SR^{f}, =NRf, =NOR^{f}, -NR^{f}R^{f}, halogéno, -CF₃, -CN, -NC, -NO₂, -N₃, -S(O)R^{f}, -S(O)₂R^{f}, -S(O)₂OR^{f}, -S(O)NR^{f}R^{f}, -S(O)₂NR^{f}R^{f}, -OS(O)R^{f}, -OS(O)₂R^{f}, -OS(O)₂OR^{f}, -OS(O)₂NR^{f}R^{f}, -C(O)R^{f}, -C(O)OR^{f}, -C(O)NR^{f}R^{f}, -C(O)N(R^{g})OR^{f}, -CN(R^{g})NR^{f}R^{f}, -OC(O)R^{f}, -OC(O)OR^{f}, -OC(O)NR^{f}R^{f}, -OCN(R^{g})NR^{f}R^{f}, -N(R^{g})C(O)R^{f}, -N(R^{g})C(S)R^{f}, -N(R^{g})S(O)₂R^{f}, -N(R^{g})C(O)OR^{f}, -N(R^{g})C(O)NR^{f}R^{f} et -N(R^{g})CN(R^{g})NR^{f}R^{f}, et
chaque R^{f} est, indépendamment les uns des autres, un atome d'hydrogène ou un radical éventuellement substitué par un ou plusieurs R^{g} identiques ou différents choisis dans le groupe comprenant les groupes alkyle en C₁ à C₆, cycloalkyle en C₃ à C₈, cycloalkylalkyle en C₄ à C₁₁, aryle en C₆ à C₁₀, arylalkyle en C₇ à C₁₆, hétéroalkyle de 2 à 6 chaînons, hétérocycloalkyle de 3 à 8 chaînons, hétérocycloalkylalkyle de 4 à 14 chaînons, hétéroaryle de 5 à 10 chaînons et hétéroarylalkyle de 6 à 16 chaînons, et chaque R^{g} est, indépendamment les uns des autres, un atome d'hydrogène, un groupe alkyle en C₁ à C₆, cycloalkyle en C₃ à C₈, cycloalkylalkyle en C₄ à C₁₁, aryle en C₆ à C₁₀, arylalkyle en C₇ à C₁₆, hétéroalkyle de 2 à 6 chaînons, hétérocycloalkyle de 3 à 8 chaînons, hétérocycloalkylalkyle de 4 à 14 chaînons, hétéroaryle de 5 à 10 chaînons et hétéroarylalkyle de 6 à 16 chaînons, éventuellement sous la forme de leurs tautomères, leurs racémates, leurs énantiomères, leurs diastéréomères et leurs mélanges, et éventuellement leurs sels pharmacologiquement acceptables.

2. Composés selon la revendication 1, de formule générale (1A) 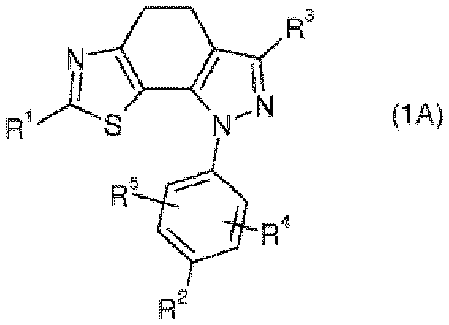 dans laquelle les substituants sont tels que définis dans la revendication 1.

3. Composés selon la revendication 1, de formule générale (1B), 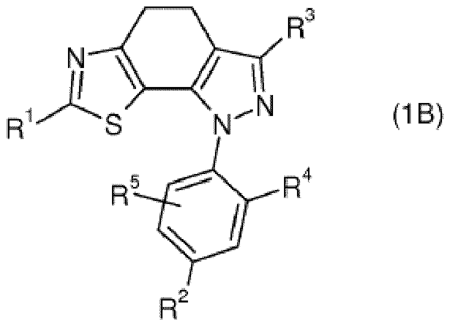 dans laquelle les substituants sont tels que définis dans la revendication 1.

4. Composés selon l'une des revendications 1 à 3, dans lesquels
R³ désigne un groupe hétéroaryle de 5 à 6 chaînons, éventuellement substitué par un ou plusieurs R^{c} et/ou R^{b} identiques ou différents.

5. Composés selon l'une des revendications 1 à 4, dans lesquels
R³ désigne un groupe pyridyle non substitué.

6. Composés ou leurs sels pharmacologiquement acceptables, selon l'une des revendications 1 à 5, comme médicament.

7. Composés ou leurs sels pharmacologiquement acceptables, selon l'une des revendications 1 à 5, pour la production d'un médicament présentant un effet antiprolifératif.

8. Préparations pharmaceutiques contenant comme substance active, un ou plusieurs composés de formule générale (1) selon l'une des revendications 1 à 7, ou leurs sels pharmacologiquement acceptables, éventuellement en combinaison avec des adjuvants et/ou véhicules habituels.

9. Utilisation de composés de formule générale (1) selon l'une des revendications 1 à 7, pour la production d'un médicament pour le traitement et/ou la prévention du cancer.

10. Préparation pharmaceutique comprenant un composé de formule générale (1) selon l'une des revendications 1 à 7, et au moins une autre substance active cytostatique ou cytotoxique différente de la formule (1), éventuellement sous la forme de leurs tautomères, leurs racémates, leurs énantiomères, leurs diastéréomères et leurs mélanges et éventuellement leurs sels pharmacologiquement acceptables.
